(19) Europäisches Patentamt
European Patent Office
Office européen des brevets

(11) EP 3 137 445 B1

(12) **EUROPÄISCHE PATENTSCHRIFT**

(45) Veröffentlichungstag und Bekanntmachung des Hinweises auf die Patenterteilung:
**02.10.2019 Patentblatt 2019/40**

(21) Anmeldenummer: **15718908.5**

(22) Anmeldetag: **29.04.2015**

(51) Int Cl.:
***C07C 45/54*** (2006.01) ***C07C 45/62*** (2006.01)
***C07C 49/04*** (2006.01) ***C07C 49/203*** (2006.01)

(86) Internationale Anmeldenummer:
**PCT/EP2015/059329**

(87) Internationale Veröffentlichungsnummer:
**WO 2015/165959 (05.11.2015 Gazette 2015/44)**

(54) **VERFAHREN ZUR HERSTELLUNG VON FARNESYLACETON**

PROCESS FOR THE PREPARATION OF FARNESYL ACETON

PROCEDE DE PREPARATION DE FARNESYL ACETONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.04.2014 EP 14166525**

(43) Veröffentlichungstag der Anmeldung:
**08.03.2017 Patentblatt 2017/10**

(73) Patentinhaber: **BASF SE**
**67056 Ludwigshafen am Rhein (DE)**

(72) Erfinder:
- **SCHELWIES, Mathias**
  **69115 Heidelberg (DE)**
- **PACIELLO, Rocco**
  **67098 Bad Dürkheim (DE)**
- **ECKHARDT, Heinz**
  **67245 Lambsheim (DE)**
- **DEHN, Martine**
  **67061 Ludwigshafen (DE)**

(56) Entgegenhaltungen:
**EP-A1- 0 806 405**
**EP-A2- 0 034 804**
**WO-A2-2010/046199**
**DE-T2- 69 103 175**
**US-A- 4 460 786**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 137 445 B1

Printed by Jouve, 75001 PARIS (FR)

**Beschreibung**

HINTERGRUND DER ERFINDUNG

Die vorliegende Erfindung betrifft ein Verfahren zur Herstellung von Farnesylaceton.

STAND DER TECHNIK

Verfahren zur Herstellung von Farnesylaceton sind im Stand der Technik bekannt. Hierbei werden Polyene mit Acetessigsäuremethylester in Gegenwart eines Katalysators umgesetzt. Der als Reaktionsprodukt entstandene β-Ketoester wird einer Decarboxylierung unterzogen unter Erhalt von Farnesylaceton.

Die erhaltenen polyenischen Ketonderivate sind Synthesezwischenprodukte für die Vitamin- und Parfumindustrie.

Die DE 69103175 T2 beschreibt ein Verfahren, bei dem eine 1,3-Butadienverbindung mit einem Acetoessigsäurealkylester in Gegenwart eines Katalysators auf Rhodium-Basis in Wasser umgesetzt wird und eine Decarboxylierung erfolgt.

Die EP 0034804 A2 beschreibt ein Verfahren zur Herstellung von gesättigten terpenoiden Ketonen, insbesondere von Hexahydrofarnesylaceton in Gegenwart von Palladium enthaltenden Katalysatoren.

Die WO 2010/046199 beschreibt ein Verfahren zur Herstellung von gamma-deltaungesättigten Ketonen in Gegenwart von Ammoniumsalzen als Katalysator.

Die US 4,621,165 beschreibt ein Verfahren, bei welchem Gemische isoprenoider Verbindungen mit Acetylessigsäuremethylester in Gegenwart von $[RhCl\text{-}(cycloocta\text{-}1,5\text{-}dien)]_2$, Triphenylphosphintrinatriumsulfonyl in einem Lösungsmittelgemisch aus Wasser und Methanol als eine Wasser-Alkohol-Mischung umgesetzt werden. Diese Gemische enthalten Farnesen als Edukt, jedoch werden zur Umsetzung große Mengen an Katalysator eingesetzt.

Die US 4,460,786 beschreibt ein Verfahren, bei welchem Farnesen mit Acetylessigsäureethylester in Gegenwart von $[RhCl\text{-}(cycloocta\text{-}1,5\text{-}dien)]_2$, Triphenylphosphintrinatriumsulfonyl in Wasser umgesetzt werden.

Bekannte Verfahren zur Herstellung von Farnesylaceton sind noch verbesserungswürdig und haben oft den Nachteil von langen Reaktionszeiten in großen, raumfüllenden Reaktoren und einem mengen- und kostenintensiven Einsatz von Katalysator(en).

Der vorliegenden Erfindung liegt daher die Aufgabe zugrunde, ein verbessertes Verfahren zur Herstellung von Farnesylaceton bereitzustellen, bei dem die zuvor genannten Nachteile vermieden werden. Insbesondere soll mit diesem Verfahren die Herstellung von Farnesylaceton in großen Mengen innerhalb kurzer Zeit möglich sein. Des Weiteren soll die Reaktionsausbeute verbessert und die für die Reaktion notwendigen Mengen an Katalysator reduziert werden können. Des Weiteren soll mit dem verbesserten Verfahren die Herstellung von Farnesylaceton kostengünstig und in Reaktoren mit kleinerer Baugröße ermöglicht werden.

ZUSAMMENFASSUNG DER ERFINDUNG

Überraschender Weise wurde nun gefunden, dass diese Aufgabe mit einem Verfahren nach Anspruch 1 gelöst wird.

Das erfindungsgemäße Verfahren zur Herstellung von Farnesylaceton erfolgt gegenüber konventionellen Verfahren in Gegenwart eines Lösungsmittel/Wasser-Gemisches mit einer Dispergierung und insbesondere bei einem Leistungseintrag im Bereich von 0,1 bis 5000 W/L.

Gegenstand der Erfindung ist daher ein Verfahren zur Herstellung von Ketoverbindungen der allgemeinen Formel (I)

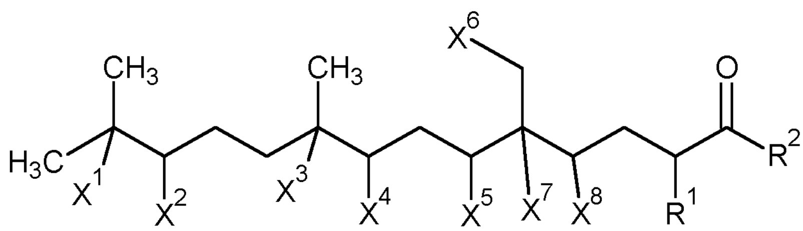

(I)

wobei

$R^1$ für Wasserstoff oder einen Rest $-C(O)OR^3$ steht, wobei

$R^3$ für $C_1$-$C_4$-Alkyl steht;
$R^2$ für $C_1$-$C_4$-Alkyl steht;
$X^1$ und $X^2$ beide für Wasserstoff oder gemeinsam für den Bindungsanteil einer Doppelbindung zwischen den Kohlenstoffatomen, an die sie gebunden sind, stehen;
$X^3$ und $X^4$ beide für Wasserstoff oder gemeinsam für den Bindungsanteil einer Doppelbindung zwischen den Kohlenstoffatomen, an die sie gebunden sind, stehen;
$X^5$, $X^6$, $X^7$ und $X^8$ für Wasserstoff stehen;
wobei eine der Kombinationen der Reste $X^5$ und $X^7$, $X^6$ und $X^7$ oder $X^7$ und $X^8$ auch für den Bindungsanteil einer Doppelbindung zwischen den Kohlenstoffatomen, an die sie gebunden sind, stehen kann,
und Isomeren und Mischungen davon,

bei dem man

a) wenigstens eine Farnesen-Verbindung der allgemeinen Formel (II)

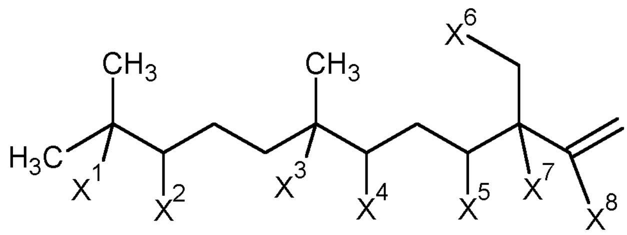

(II)

worin

$X^1$ und $X^2$ gemeinsam für den Bindungsanteil einer Doppelbindung zwischen den Kohlenstoffatomen, an die sie gebunden sind, stehen;
$X^3$ und $X^4$ gemeinsam für den Bindungsanteil einer Doppelbindung zwischen den Kohlenstoffatomen, an die sie gebunden sind, stehen;
$X^5$ und $X^7$ gemeinsam für den Bindungsanteil einer Doppelbindung zwischen den Kohlenstoffatomen, an die sie gebunden sind, stehen unter der Maßgabe, dass $X^6$ für Wasserstoff steht; oder
$X^6$ und $X^7$ gemeinsam für den Bindungsanteil einer Doppelbindung zwischen den Kohlenstoffatomen, an die sie gebunden sind, stehen unter der Maßgabe, dass $X^5$ für Wasserstoff steht, und
$X^8$ für Wasserstoff steht;

mit einem β-Ketoester der allgemeinen Formel (III)

$$R^2\text{-CO-CH}_2\text{-R}^1 \qquad \text{(III)}$$

worin

$R^1$ für einen Rest -C(O)O$R^3$ steht, wobei $R^3$ für $C_1$-$C_4$-Alkyl steht;

in Gegenwart eines Katalysators und eines Lösungsmittel/Wasser-Gemischs einer Umsetzung unterwirft, wobei man das Reaktionsgemisch einer Dispergierung mit mindestens einem Mischer bei einer Reynoldszahl von größer als $10^4$ unterzieht,

unter Erhalt einer Verbindung der Formel (I-a),

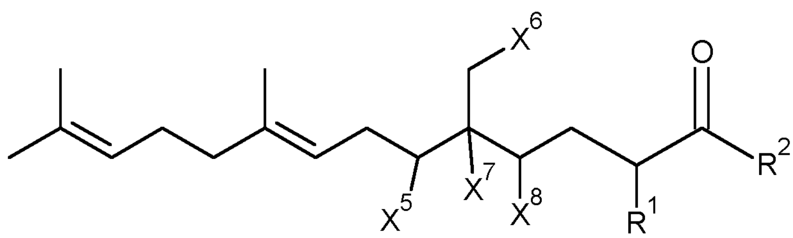

(I-a)

worin

$R^1$ für einen Rest -C(O)OR$^3$ steht,
wobei $R^3$ für $C_1$-$C_4$-Alkyl steht;
$R^2$ für $C_1$-$C_4$-Alkyl steht;
eine der Kombinationen der Reste $X^5$ und $X^7$, $X^6$ und $X^7$ oder $X^7$ und $X^8$ gemeinsam für den Bindungsanteil einer Doppelbindung zwischen den Kohlenstoffatomen, an die sie gebunden sind, steht, und die übrigen Reste $X^5$, $X^6$, $X^7$ und $X^8$ für Wasserstoff stehen;
und Isomere und Mischungen davon;

b) gegebenenfalls das in Schritt a) erhaltene Reaktionsgemisch einer Decarboxylierung unterzieht, wobei eine Verbindung der Formel (I-b),

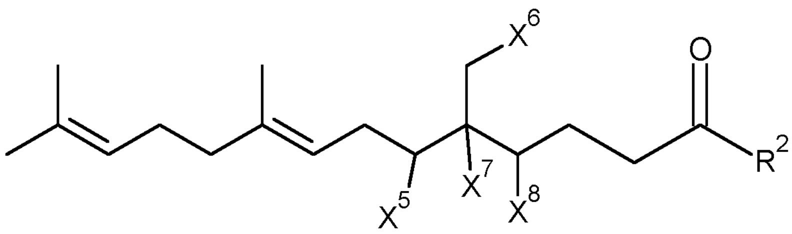

(I-b)

worin

$R^2$ für $C_1$-$C_4$-Alkyl steht; und
eine der Kombinationen der Reste $X^5$ und $X^7$, $X^6$ und $X^7$ oder $X^7$ und $X^8$ gemeinsam für den Bindungsanteil einer Doppelbindung zwischen den Kohlenstoffatomen, an die sie gebunden sind, steht, und die übrigen Reste $X^5$, $X^6$, $X^7$ und $X^8$ für Wasserstoff stehen;

und Isomere und Mischungen davon erhalten wird,

c) gegebenenfalls das in Schritt b) erhaltene Reaktionsgemisch einer Hydrierung unterzieht, wobei eine Verbindung der Formel (I-c)

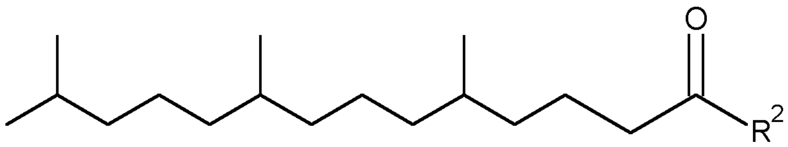

(I-c)

worin

$R^2$ für $C_1$-$C_4$-Alkyl steht;

erhalten wird;

wobei das Lösungsmittel des Lösungsmittel/Wasser-Gemisches in Schritt a) ausgewählt ist unter Ethanol, n-Propanol, iso-Propanol, n-Butanol, t-Amylalkohol, Monoethylenglycol, Diethylenglycol, Tetrahydrofuran, Dioxan, Methyl-tert-butylether, 1,2-Dimethoxyethan, Diethylenglykoldimethylether, Triethylenglykoldimethylether, Acetonitril, Aceton, Essigsäuremethylester, Essigsäureethylester, Dimethylformamid, N-Methylpyrrolidon und Mischungen hiervon.

Ein weiterer Gegenstand der Erfindung ist die Verwendung von Farnesylaceton, erhalten nach dem erfindungsgemäßen Verfahren, zur Herstellung von Vitamin E, Isophytol, Dehydro-Isophytol, Hexahydrofarnesylaceton, Tetrahydrofarnesylaceton.

DETAILLIERTE BESCHREIBUNG DER ERFINDUNG

Im Rahmen der vorliegenden Erfindung steht der Begriff "Alkyl" für geradkettiges oder verzweigtes $C_1$-$C_6$-Alkyl und besonders bevorzugt für $C_1$-$C_4$-Alkyl. Alkyl steht insbesondere für Methyl, Ethyl, n-Propyl, Isopropyl, n-Butyl, Isobutyl

(2-Methylpropyl), sec.-Butyl (1-Methylpropyl), tert.-Butyl (1,1-Dimethylethyl), n-Pentyl oder n-Hexyl. Speziell steht Alkyl für Methyl, Ethyl, n-Propyl, Isopropyl oder Isobutyl, insbesondere für Methyl.

Sofern im Folgenden nicht genauer angegeben, bezeichnet der Begriff "Farnesen-Verbindung" im Rahmen der Erfindung α-Farnesen (3,7,11-Trimethyl-1,3,6,10-dodecatetraen) in Reinform, β-Farnesen (7,11-Dimethyl-3-methylen-1,6,10-dodecatrien) in Reinform sowie ein Gemisch aus α-Farnesen und β-Farnesen jedweder Zusammen-Zusammensetzung. Der Begriff Farnesen-Verbindung bezeichnet weiterhin Mischungen der Isomere von α-Farnesen, Mischungen der Isomere von β-Farnesen oder ein Isomer von α-Farnesen oder β-Farnesen in Reinform. Von α-Farnesen existieren vier Isomere, nämlich (3E,6E)-3,7,11-Trimethyl-1,3,6,10-dodecatetraen, (3Z,6Z)-3,7,11-Trimethyl-1,3,6,10-dodecatetraen,(3Z,6E)-3,7,11-Trimethyl-1,3,6,10-dodecatetraen und (3E,6Z)-3,7,11-Trimethyl-1,3,6,10-dodecatetraen. Von β-Farnesen existieren zwei Isomere, nämlich (6E)-7,11-Dimethyl-3-methylen-1,6,10-dodecatrien und (6Z)-7,11-Dimethyl-3-methylen-1,6,10-dodecatrien. Sofern im Folgenden von β-Farnesen der Formel (IIA) die Rede ist, wird nur ein Isomer abgebildet.

Sofern im Folgenden nicht genauer angegeben, bezeichnet der Begriff "Farnesylaceton" der Formel (I-aA) im Rahmen der Erfindung 6,10,14-Trimethylpentadeca-5,9,13-trien-2-on in Reinform, 6,10,14-Trimethylpentadeca-6,9,13-trien-2-on in Reinform, 10,14-Dimethyl-6-methylenpentadeca-9,13-trien-2-on in Reinform sowie eine Mischung aus den Isomeren 6,10,14-Trimethylpentadeca-5,9,13-trien-2-on, 6,10,14-Trimethylpentadeca-6,9,13-trien-2-on und 10,14-Dimethyl-6-methylenpentadeca-9,13-trien-2-on in jedweder Zusammensetzung. Der Begriff Farnesylaceton der Formel (I-aA) bezeichnet weiterhin Mischungen von 6,10,14-Trimethylpentadeca-5,9,13-trien-2-on oder ein Isomer davon in Reinform, Mischungen von 6,10,14-Trimethylpentadeca-6,9,13-trien-2-on oder ein Isomer davon in Reinform sowie Mischungen von 10,14-Dimethyl-6-methylenpentadeca-9,13-trien-2-on oder ein Isomer davon in Reinform. Von 6,10,14-Trimethylpentadeca-5,9,13-trien-2-on existieren vier Isomere, nämlich (5E,9E)-6,10,14-Trimethylpentadeca-5,9,13-trien-2-on (E,E-Farnesylaceton), (5Z,9Z)-6,10,14-Trimethylpentadeca-5,9,13-trien-2-on (Z,Z-Farnesylaceton), (5Z,9E)-6,10,14-Trimethylpentadeca-5,9,13-trien-2-on (Z,E-Farnesylaceton) und (5E,9Z)-6,10,14-Trimethylpentadeca-5,9,13-trien-2-on. Von 6,10,14-Trimethylpentadeca-5,9,13-trien-2-on existieren vier Isomere, nämlich (6E,9E)-6,10,14-Trimethylpentadeca-6,9,13-trien-2-on, (6Z,9Z)-6,10,14-Trimethylpentadeca-6,9,13-trien-2-on, (6E,9Z)-6,10,14-Trimethylpentadeca-6,9,13-trien-2-on und (6Z,9E)-6,10,14-Trimethylpentadeca-6,9,13-trien-2-on. Von 10,14-Dimethyl-6-methylenpentadeca-9,13-trien-2-one existieren zwei Isomere, nämlich (9E)-10,14-Dimethyl-6-methylenpentadeca-9,13-trien-2-on und (9Z)-10,14-Dimethyl-6-methylenpentadeca-9,13-trien-2-on.

Im Rahmen der vorliegenden Erfindung werden die Begriffe "trans" und "E" synonym verwendet, insbesondere als "trans-β-Farnesen" und "β-E-Farnesen", falls nicht anders angegeben.

Sofern im Folgenden von Farnesylaceton der Formeln (I-aA) oder (I-aA1) die Rede ist, wird nur ein Isomer abgebildet.

Im Rahmen der vorliegenden Erfindung wird unter dem Ausdruck "Ketoverbindungen der allgemeinen Formel (I), und Isomere und Mischungen davon" Ketoverbindungen der allgemeinen Formel (I) und Isomere der Verbindungen der allgemeinen Formel (I) und Mischungen der Verbindungen der allgemeinen Formel (I) und deren Isomere verstanden.

Im Rahmen der vorliegenden Erfindung wird unter Dispergieren das Mischen von mindestens zwei Stoffen, die sich ohne Energieeintrag nicht oder kaum ineinander lösen, verstanden. Beim Dispergieren wird ein Stoff (disperse Phase) in einem anderen Stoff (kontinuierliche Phase) verteilt. Im vorliegenden Fall ist das Ziel die Herstellung einer Emulsion (flüssig/flüssig). Zwischen den zwei Phasen bildet sich an der Grenzfläche eine Grenzflächenspannung, die verhindert, dass sich spontan eine Dispersion ausbildet. Durch den Energieeintrag beim Dispergieren werden die Flüssigkeitstropfen verkleinert, so dass es zu einem Ausgleich im Bereich der Phasengrenzen kommt. Dadurch wird eine momentane Stabilität der Dispersion bewirkt. Das Ziel einer Dispergierung ist eine Vergrößerung von Phasengrenzflächen, damit bspw. chemische Reaktionen schneller ablaufen können.

Im Rahmen der vorliegenden Erfindung wird unter einem Mischer ein Rotor-Stator-Rührsystem, ein Rührer/Rührorgan ausgewählt unter Kreuzbalkenrührern, Gitterrührern, Blattrührern, Ankerrührern, Wendelrührern, MIG-Rührern, Scheibenrührern, Propellerrührern, Impellerrührern, Halbmondrührern und Kombinationen hiervon verstanden.

Das erfindungsgemäße Verfahren weist die folgenden Vorteile auf:

- Der benötigte Leistungseintrag für eine Dispergierung ist geringer im Vergleich zu konventionellen Verfahren,
- Die benötigte Einsatz an Katalysator für die Umsetzung ist geringer im Vergleich zu konventionellen Verfahren,
- Die Raum-Zeit-Ausbeute ist im Vergleich zu konventionellen Verfahren größer,
- Der Umsatz des Eduktes ist bei dem erfindungsgemäßen Verfahren in Gegenwart eines Ethanol/Wasser-Gemisches im Vergleich zu einem konventionellen Verfahren mit Wasser mehr als doppelt so groß.

Schritt a)

Geeignete Farnesen-Verbindungen für den Einsatz in Schritt a) können wenigstens eine β-Farnesen-Verbindung der Formel (IIA)

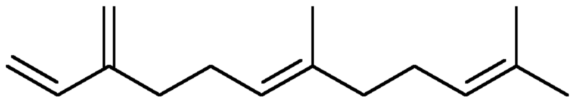

(IIA)

enthalten. In einer bevorzugten Ausführungsform enthält die Farnesen-Verbindung der Formel (II) wenigstens eine β-Farnesen-Verbindung der Formel (IIA) in einer Menge von wenigstens 70 Gew.-%, insbesondere von wenigstens 80 Gew.-%, ganz besonders bevorzugt von wenigstens 90 Gew.-%, bezogen auf das Gesamtgewicht der Farnesen-Verbindung der Formel (II).

Bevorzugt ist die β-Farnesen-Verbindung β-E-Farnesen ((6E)-7,11-Dimethyl-3-methylen-1,6,10-dodecatrien).

In einer weiteren bevorzugten Ausführungsform wird in Schritt a) eine Mischung aus einer α-Farnesen-Verbindung und einer β-Farnesen-Verbindung eingesetzt. Geeignet ist eine Mischung aus α-Farnesen-Verbindung und β-Farnesen-Verbindung, vorzugsweise β-E-Farnesen, in jedweder Zusammensetzung.

Geeignete Farnesen-Verbindungen für den Einsatz in Schritt a) können weiterhin wenigstens eine α-Farnesen-Verbindung, in im Wesentlichen reiner Form enthalten. Unter im Wesentlichen reiner Form wird verstanden, dass das für den Einsatz in Schritt a) eingesetzte Farnesen-Verbindung vorzugsweise zu wenigstens 70 Gew.-%, besonders bevorzugt zu wenigstens 80 Gew.-%, insbesondere zu wenigstens 90 Gew.-%, bezogen auf das Gesamtgewicht der Farnesen-Verbindung, aus einer α-Farnesen-Verbindung der allgemeinen Formel(II) besteht.

Vorzugsweise wird in Schritt a) als β-Ketoester der allgemeinen Formel (III) Acetessigsäuremethylester oder Acetessigsäureethylester eingesetzt.

Die Umsetzung in Schritt a) erfolgt in Gegenwart eines Katalysators und eines Lösungsmittel/Wasser-Gemischs, wobei das Reaktionsgemisch einer Dispergierung mit mindestens einem Mischer bei einer Reynoldszahl von größer als $10^4$ unterzogen wird.

Als Katalysator eignen sich beispielsweise die in US 4,621,165 oder in EP 0441708 beschriebenen Katalysatoren.

Nach einer bevorzugten Ausführungsform wird der Katalysator in situ hergestellt. Gewünschtenfalls kann der Katalysator jedoch auch separat hergestellt und nach üblichen Verfahren isoliert werden. Zur in situ Herstellung des Katalysators kann man wenigstens eine Verbindung oder einen Komplex eines Übergangsmetalls der 9. Gruppe des Periodensystems mit einem wasserlöslichen Phosphin umsetzen. Ein so hergestellter Katalysator vermag sich in der Regel unter den Reaktionsbedingungen in Wasser zu lösen. Vorzugsweise ist die Verbindung oder der Komplex eines Übergangsmetalls der 9. Gruppe des Periodensystems eine Rhodium-Verbindung oder ein Rhodiumkomplex.

Geeignete Rhodium-Verbindungen oder -komplexe sind z. B. Rhodium(I)-, Rhodium(II)- und Rhodium (III)-salze, wie Rhodium(III)-chlorid, Rhodium(III)-bromid, Rhodium(III)-nitrat, Rhodium(III)-sulfat, Rhodium(II)- oder Rhodium(III)-oxid, Rhodium(II)- oder Rhodium(III)-acetat, Rhodium(II)- oder Rhodium(III)-carboxylat, $Rh(CH_3COCH_2COCH_3)_3$, Rhodiumdicarbonylacetylacetonat, $[RhCl(cycloocta\text{-}1,5\text{-}dien)]_2$, $[RhCl(CO)_2]_2$ und $RhCl_3(C_2H_5NH_2)_3$. Vorzugsweise wird $[RhCl(cycloocta\text{-}1,5\text{-}dien)]_2$ eingesetzt.

Geeignete Phosphin-Verbindungen sind Alkalimetall, Erdalkalimetall, Ammonium oder quaternäre Ammoniumsalze von (para-Sulfonatophenyl)-diphenylphosphin; (meta-Sulfonato-para-methylphenyl)-di(para-methylphenyl)phosphin; (meta-Sulfonato-para-methoxyphenyl)-di(para-methoxyphenyl)phosphin; (meta-Sulfonato-para-chlorphenyl)-di(para-chlorophenyl)phosphin; di(para-Sulfonatophenyl)phenylphosphin; di(meta-Sulfonato-para-methylphenyl)-(para-methylphenyl)phosphin; di(meta-Sulfonato-para-methoxymethyl)-(para-methoxyphenyl)phosphin; di(meta-Sulfonato-para-chlorophenyl)-(para-chlorphenyl) phosphin; tri(para-Sulfonatophenyl)phosphin; tri(meta-Sulfonatophenyl)phosphin, tri(meta-Sulfonato-para-methylphenyl)phosphin; tri(meta-Sulfonato-para-methoxyphenyl)phosphin; tri(meta-Sulfonato-para-methoxyphenyl)phosphin; tri(meta-Sulfonato-para-chlorophenyl)phosphin;(ortho-Sulfonato-para-methylphenyl)-(meta-Sulfonato-para-methyl)-(meta, meta'-disulfonato-para-methyl)phosphin; und (meta-Sulfonatophenyl)-(meta-sulfonatopara-chlorphenyl)-(meta, meta'-disulfonato-para-chlorophenyl)phosphin. Vorzugsweise wird Tri-(natrium-meta-sulfonatonatophenyl)-phosphin verwendet.

Die Phosphin-Verbindung und die Rhodium-Verbindung bzw. der Rhodium-Komplex werden üblicherweise in einem molaren Verhältnis von etwa 1 : 1 bis 50 : 1, vorzugsweise von 1:1 zu 1 bis 30 : 1, insbesondere von 1 : 1 bis 25 : 1, eingesetzt.

Schritt a) wird üblicherweise unter Schutzgasatmosphäre durchgeführt.

Vorzugsweise umfasst das Verfahren zur Herstellung von Farnesylaceton der Formel (I-aA),

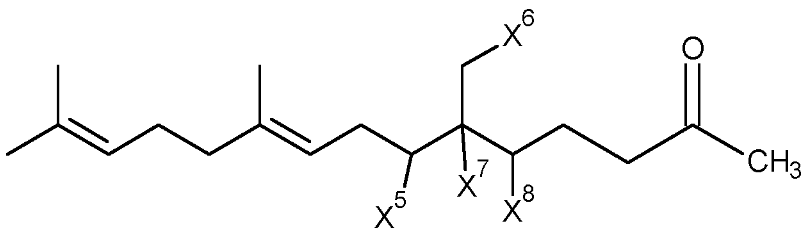

(I-aA)

worin eine der Kombinationen der Reste $X^5$ und $X^7$, $X^6$ und $X^7$ oder $X^7$ und $X^8$ gemeinsam für den Bindungsanteil einer Doppelbindung zwischen den Kohlenstoffatomen, an die sie gebunden sind, steht, und die übrigen Reste $X^5$, $X^6$, $X^7$ und $X^8$ für Wasserstoff stehen, und Isomere und Mischungen davon, die Schritte a) und b).

Vorzugsweise umfasst das Verfahren zur Herstellung von Hexahydrofarnesylaceton (6,10,14-Trimethyl-2-pentadecanon) der Formel (I-bB),

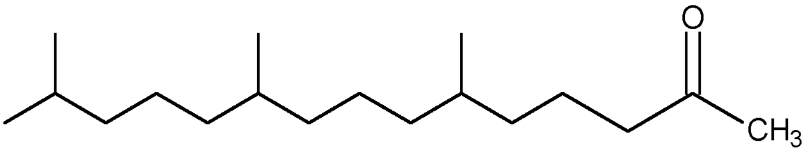

(I-bB)

die Schritte a), b), und c).

Erfindungsgemäß ist das Lösungsmittel des Lösungsmittel/Wasser-Gemisches ausgewählt unter Ethanol, n-Propanol, iso-Propanol, n-Butanol, t-Amylalkohol, Monoethylenglycol, Diethylenglycol, Tetrahydrofuran (THF), Dioxan, Methyl-tert-butylether, 1,2-Dimethoxyethan, Diethylenglykoldimethylether, Triethylenglykoldimethylether, Acetonitril, Aceton, Essigsäuremethylester, Essigsäureethylester, Dimethylformamid, N-Methylpyrrolidon und Mischungen hiervon.

Vorzugsweise liegt das Lösungsmittel/Wasser-Gemisch in einem Volumenverhältnis von vorzugsweise 1 : 5 bis 5 : 1, besonders bevorzugt von 2 : 1 bis 1 : 2, ganz besonders bevorzugt von 1 : 1, jeweils gerechnet als Reinstoff, vor.

In der Regel wird Schritt a) in Gegenwart einer Base durchgeführt. Geeignete Basen sind Alkali- und Erdalkalimetallhydroxide, Alkali- und Erdalkalimetallhydrogencarbonate, Alkali- und Erdalkalimetallcarbonate, Akalimetallphosphate oder Alkalimetallalkanolate von $C_1$-$C_6$-Alkanolen durchgeführt. Vorzugsweise werden als Base Alkalimetallhydroxide wie Natriumhydroxid oder Kaliumhydroxid, Alkalimetallcarbonate wie Natriumcarbonat oder Kaliumcarbonat, Alkalimetallhydrogencarbonate wie Natriumhydrogencarbonat oder Kaliumhydrogencarbonat, Alkalimetallphosphate wie $Na_3PO_4$ oder $K_3PO_4$ oder Alkalimetallalkanolate wie Natriummethanolat, Natriumethanolat, Natriumpropanolat, Natriumbutanolat, Kaliummethanolat, Kaliumethanolat oder Kaliumbutanolat eingesetzt.

Bevorzugt sind insbesondere Natriumcarbonat, Kaliumcarbonat und Natriumphosphat. Ganz besonders bevorzugt wird Natriumcarbonat eingesetzt.

In einer speziellen Ausführungsform wird Schritt a) in Gegenwart von Natriumcarbonat durchgeführt.

Vorzugsweise erfolgt die Dispergierung in Schritt a) bei einem Leistungseintrag im Bereich von vorzugsweise 0,1 bis 5000 W/L, besonders bevorzugt von 10 bis 600 W/L, ganz besonders bevorzugt von 20 bis 100 W/L.

Im Rahmen der vorliegenden Erfindung wird der Leistungseintrag der eingesetzten Rührer wie nachfolgend beschrieben experimentell bestimmt, wie insbesondere in den Beispielen E1 und E2 dargestellt.

Für Rotor-Stator-Rührsysteme:

Für ein Rotor-Stator-Rührsystem wird in einem adiabaten Rührgefäß eine Anfangsmenge eines zu rührenden Systems mit einer oder mehreren Phase(n) und einer Dichte vorgelegt und das Rotor-Stator-Rührsystem in dem Rührgefäß angeordnet. Die Anfangstemperatur des zu rührenden Systems wird gemessen, das Rotor-Stator-Rührsystem gestartet und die Temperaturänderung mit dem Einbringen von Scherkräften in das zu rührenden System durch das Rotor-Stator-Rührsystem über eine Rührzeit t bei einer Drehzahl n des Rotor-Stator-Rührsystems gemessen. Zudem wird die Endtemperatur des zu rührenden Systems gemessen. Mit den ermittelten Daten wird der spezifische Leistungseintrag in das zu rührende System berechnet. Die nachfolgenden Berechnungen werden am Beispiel von Wasser als zu rührendes System durchgeführt.

Der Leistungseintrag wird nach Formel 1 berechnet:

$$\text{Formel 1:}\quad P = [(mW_{quer} * cpW_{quer} * \Delta T) + (\Delta mW * \Delta H_{quer})]/\ t$$

wobei P der volumenspezifische Leistungseintrag in Watt, $mW_{quer}$ die mittlere Wassermenge in kg, $cpW_{quer}$ die mittlere Wärmekapazität von Wasser in kJ/(kg*K), $\Delta T$ die Differenz zwischen Anfangs- und Endtemperatur des Wassers in °C, $\Delta mW$ die Differenz zwischen Anfangs- und Endwassermenge in kg, $\Delta H_{quer}$ die mittlere Verdampfungsenthalpie von Wasser in kJ/kg und t die Rührzeit in s ist.

Mit dem in der Formel 1 bestimmten Leistungseintrag P wird die dimensionslose Newton-Leistungskennzahl Ne berechnet nach Formel 2:

$$\text{Formel 2:} \quad Ne = P / (\rho W^* n^3 * d_2^5)$$

wobei P der volumenspezifische Leistungseintrag in Watt, pW die Dichte von Wasser in $kg/m^3$, n die Rührerdrehzahl in $s^{-1}$ und $d_2$ der Rührorgandurchmesser in m ist.

In einem turbulenten Strömungsbereich in Rührersystemen ist die Ne-Zahl nahezu konstant. Die dimensionslose Reynolds-Zahl wird mit nachfolgender Formel 3 ermittelt.

$$\text{Formel 3:} \quad Re = n * d_2^2 * (\rho/\eta)$$

wobei n die Rührerdrehzahl in $s^{-1}$, $d_2$ der Rührorgandurchmesser in m, $\rho$ die Dichte des zu rührenden Systems in $kg/m^3$ und $\eta$ die dynamische Viskosität des zu rührenden Systems in Pa·s ist.

Für Strömungssysteme in welchen Rührer eingesetzt werden, kann eine turbulente Strömung in dem Bereich von Reynoldszahlen von größer als 1000 angenommen werden.

Mit nachfolgender Formel 4 wird der mit der Formel 1 für Wasser bestimmte Leistungseintrag auf ein spezifisches zu rührenden Systems mit einer spezifischen Dichte übertragen und somit die spezifische Leistung Ps bestimmt:

$$\text{Formel 4:} \quad Ps/P = Ne * (\rho s^* n^3 * d_2^5) / P$$

wobei Ps die spezifische Leistung in Watt, P der volumenspezifische Leistungseintrag in Watt, Ne die dimensionslose Newton-Leistungskennzahl, $\rho s$ die spezifische Dichte des spezifischen zu rührenden Systems in $kg/m^3$ und $d_2$ der Rührorgandurchmesser in m ist.

Für Systeme mit Rührorganen (Rührer-Systeme):

Ein Rührorgan kann beispielsweise ein Halbmondrührer sein. Bei der gegebenen Drehzahl n in $min^{-1}$ wird im Reaktionsprodukt das Drehmoment M in $Ncm^{-1}$ gemessen.

Die Leistung berechnet sich mit nachfolgender Formel 5 wie folgt:

$$\text{Formel 5:} \quad Ps = M *2*pi*n$$

wobei Ps die spezifische Leistung in Watt, M das Drehmoment in $Ncm^{-1}$ und n die Drehzahl in $min^{-1}$ ist.

Der volumenspezifische Leistungseintrag Ps in Watt wird berechnet mit Bezug auf das Volumen des zu rührenden Systems.

Im Rahmen der vorliegenden Erfindung wird unter Rühren insbesondere Mischen, Durchmischen, Homogenisieren, Dispergieren, Suspendieren, Emulgieren verstanden.

Vorzugsweise ist ein Rührer auch als Rührorgan bezeichnet, ausgewählt unter Kreuzbalkenrührern, Gitterrührern, Blattrührern, Ankerrührern, Wendelrührern, MIG-Rührern, Scheibenrührern, Propellerrührern, Impellerrührern, Halbmondrührern und Kombinationen hiervon.

Vorzugsweise erfolgt die Dispergierung in Schritt a) mit einem Rührer bei einer Umfangsgeschwindigkeit im Bereich von 1 bis 80 m/s, vorzugsweise im Bereich von 1,8 bis 30 m/s.

Vorzugsweise erfolgt die Dispergierung in Schritt a) bei einer Temperatur im Bereich von vorzugsweise 50 bis 200 °C, bevorzugt von 60 bis 150 °C, besonders bevorzugt von 70 bis 120 °C, ganz besonders bevorzugt im Bereich von 80 bis 100 °C.

Nach üblichen Verfahren kann man die in Schritt a) erhaltene Verbindung der Formel (I-a), und Isomere und Mischungen davon aus dem Reaktionsgemisch isolieren. In der Regel kann das in Schritt a) erhaltene Reaktionsgemisch ohne weitere Reinigung für die Folgeumsetzungen eingesetzt werden.

Schritt b)

In einer ersten Ausführungsform umfasst Schritt b) die Alkyldecarboxylierung oder Verseifung/Decarboxylierung des in Schritt a) erhaltenen Reaktionsgemischs bei erhöhten Temperaturen.

In einer zweiten Ausführungsform umfasst Schritt b) die Alkyldecarboxylierung oder Verseifung/Decarboxylierung der Verbindung der Formel (I-a), und Isomere und Mischungen davon bei erhöhten Temperaturen in einem Lösungsmittel/Wasser-Gemisch. Geeignete Lösungsmittel /Wasser-Gemische sind die in Schritt a) genannten Lösungsmittel/Wasser-Gemische.

Üblicherweise wird Schritt b) in Gegenwart eines Salzes durchgeführt. Geeignete Salze umfassen Alkali-und Erdalkalimetallcarbonate wie Natriumcarbonat oder Kaliumcarbonat, Alkalimetallhalogenide wie Lithiumchlorid, Lithiumiodid, Natriumchlorid, Natriumbromid oder Natriumiodid, Kaliumchlorid, Erdalkalimetallhalogenide und Alkalimetallphosphate wie Natriumphosphat oder Kaliumphosphat.

Üblicherweise wird Schritt b) bei einer Temperatur im Bereich von 50 bis 250 °C, vorzugsweise von 100 bis 200 °C, insbesondere von 120 bis 200 °C durchgeführt.

Es kann vorteilhaft sein, Schritt b) in einem Autoklaven durchzuführen.

Eine bevorzugte Ausführungsform des erfindungsgemäßen Verfahrens ist dadurch gekennzeichnet, dass die Schritte a) und b) einstufig (Eintopfreaktion) durchgeführt werden, d. h. ohne Isolierung der Verbindung der Formel (I-a), Isomere und Mischungen davon.

Schritt c)

Geeignete Hydrierungskatalysatoren sind geträgerte Palladium-Katalysatoren. Als Träger eignet sich eine Vielzahl von Materialien, beispielsweise Aluminiumoxid, keramische Trägermaterialien oder Kohlenstoff bzw. Graphit. Trägermaterialien für diese Katalysatoren sind dem Fachmann bekannt und werden in der Regel in feinverteilter Form verwendet, die gegebenenfalls zu Pellets verpresst sein können. Besonders bevorzugt wird Kohlenstoff, insbesondere Aktivkohle, als Trägermaterial eingesetzt. Ebenfalls bevorzugt ist die Verwendung von Aluminiumoxid als Trägermaterial. Ganz besonders bevorzugt wird Palladium auf Kohle (10 %) eingesetzt.

Die Hydrierung erfolgt üblicherweise in einem Lösungsmittel. Geeignete Lösungsmittel umfassen cyclische Ether wie Tetrahydrofuran (THF) oder Dioxan oder Alkanole wie beispielsweise Methanol oder Ethanol.

Schritt c) wird üblicherweise bei einer Temperatur von 20 bis 100 °C durchgeführt.

Die Umsetzung erfolgt vorzugsweise in einem Autoklaven. Die Hydrierung erfolgt üblicherweise bei einem Wasserstoffdruck von 0,5 bis 20 bar, bevorzugt von 0,5 bis 15 bar, und besonders bevorzugt bei 1 bis 12 bar.

Bezüglich näherer Einzelheiten über katalytische Hydrierungen von Polyen-Verbindungen wird auf die EP 34804 verwiesen.

Eine spezielle Ausführungsform des erfindungsgemäßen Verfahrens ist ein Verfahren zur Herstellung von Farnesylaceton der Formel (I-aA),

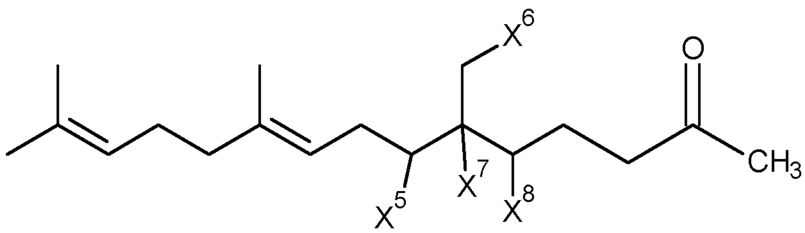
(I-aA)

Isomeren und Mischungen davon,
worin
eine der Kombinationen der Reste $X^5$ und $X^7$, $X^6$ und $X^7$ oder $X^7$ und $X^8$ gemeinsam für den Bindungsanteil einer Doppelbindung zwischen den Kohlenstoffatomen, an die sie gebunden sind, steht, und die übrigen Reste $X^5$, $X^6$, $X^7$ und $X^8$ für Wasserstoff stehen,
wobei das Verfahren die Schritte a) und b) umfasst.

Speziell bevorzugt ist ein Verfahren zur Herstellung von Farnesylaceton der Formel (I-aA.1)

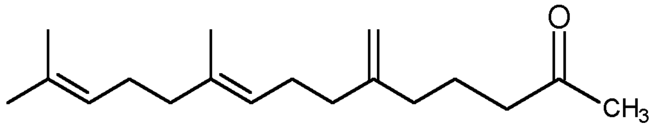

(I-aA.1)

und Isomeren und Mischungen davon, umfassend die Schritte a) und b) des erfindungsgemäßen Verfahrens, wobei in Schritt a) die Farnesenverbindung der Formel (II) zu wenigstens 70 Gew.-%, vorzugsweise zu wenigstens 80 Gew.-% und insbesondere zu wenigstens 90 Gew.-% β-E-Farnesen ist und der β-Ketoester der allgemeinen Formel (III) Acetessigsäuremethylester ist.

Gemäß einer weiteren bevorzugten Ausführungsform wird in Schritt a) als Farnesenverbindung der Formel (II) ein Gemisch aus β-Farnesen und α-Farnesen und als β-Ketoester der allgemeinen Formel (III) Acetessigsäuremethylester eingesetzt. Die Umsetzung erfolgt in Gegenwart von Natriumcarbonat.

Mit Hilfe der Schritte a) und b) des erfindungsgemäßen Verfahrens ist es möglich, Farnesylaceton, das ein wichtiges Zwischenprodukt für die Herstellung von Hexahydrofarnesylaceton ist, einfach und in großtechnischem Maßstab auf vorteilhafte Weise herzustellen.

Speziell bevorzugt ist ebenfalls ein Verfahren zur Herstellung von Hexahydrofarnesylaceton (6,10,14-Trimethyl-2-pentadecanon) der Formel (I-bB.1),

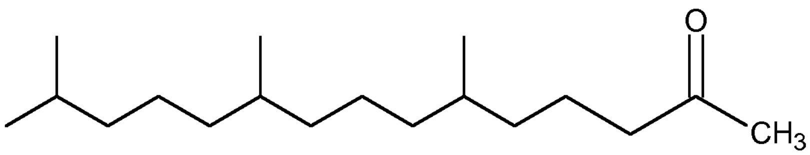

(I-bB.1)

umfassend die Schritte a), b) und c) des erfindungsgemäßen Verfahrens, wobei in Schritt a) die Farnesenverbindung der Formel (II) β-E-Farnesen ist und die β-Ketoester der allgemeinen Formel (111) Acetessigsäuremethylester ist. Gemäß einer weiteren bevorzugten Ausführungsform ist die in Schritt a) eingesetzte Farnesenverbindung der Formel (II) ein Gemisch aus β-Farnesen und α-Farnesen und der β-Ketoester der allgemeinen Formel (III) ist Acetessigsäuremethylester.

Mit Hilfe der Schritte a), b) und c) des erfindungsgemäßen Verfahrens ist es möglich Hexahydrofarnesylaceton, das ein wichtiges Zwischenprodukt für die Herstellung von Phytol bzw. Isophytol und damit für die Herstellung von Vitamin E ist, einfach und in großtechnischem Maßstab auf vorteilhafte Weise herzustellen.

## FIGURENBESCHREIBUNG UND BEISPIELE

Die Erfindung wird nachstehend durch die Figuren A1 bis C1 und den zugehörigen Beispielen A1 bis E1 näher erläutert.

Fig. A1 zeigt den Reaktionsverlauf eines Verfahrens nach dem Stand der Technik bei Verwendung eines Lösungsmittelgemisches von Methanol und Wasser in einem Volumenverhältnis von 3 : 1, jeweils gerechnet als Reinstoff, bei einer Durchmischung mit einer Rührerdrehzahl von 500 U/min und einem Leistungseintrag von 11 W/L,

Fig. A2 zeigt den Reaktionsverlauf eines Verfahrens nach dem Stand der Technik bei Verwendung eines Lösungsmittelgemisches von Methanol und Wasser in einem Volumenverhältnis von 1 : 3, jeweils gerechnet als Reinstoff, bei einer Durchmischung mit einer Rührerdrehzahl von 500 U/min und einem Leistungseintrag von 11 W/L,

Fig. A3 zeigt den Reaktionsverlauf eines Verfahrens nach dem Stand der Technik bei Verwendung eines Lösungsmittelgemisches von Methanol und Wasser in einem Volumenverhältnis von 1 : 3, jeweils gerechnet als Reinstoff, bei einer Durchmischung mit einer Rührerdrehzahl von 20000 U/min und einem Leistungseintrag von 662 W/L,

Fig. A4 zeigt den Reaktionsverlauf eines Verfahrens nach dem Stand der Technik bei Verwendung eines Lösungsmittelgemisches von Methanol und Wasser in einem Volumenverhältnis von 1 : 1, jeweils gerechnet als Reinstoff, bei einer Durchmischung mit einer Rührerdrehzahl von 20000 U/min und einem Leistungseintrag von 662 W/L,

Fig. B1 zeigt den Reaktionsverlauf des erfindungsgemäßen Verfahrens bei Verwendung eines Lösungsmittelgemisches von Ethanol und Wasser in einem Volumenverhältnis von 1 : 1 bei einer Durchmischung mit einer Rührerdrehzahl von 500 U/min und einem Leistungseintrag von 11 W/L,

Fig. B2 zeigt den Reaktionsverlauf des erfindungsgemäßen Verfahrens bei Verwendung eines Lösungsmittelgemisches von Ethanol und Wasser in einem Volumenverhältnis von 1 : 1, jeweils gerechnet als Reinstoff bei einer Durchmischung mit einer Rührerdrehzahl von 20000 U/min und einem Leistungseintrag von 662 W/L,

Fig. B3 zeigt den Reaktionsverlauf des erfindungsgemäßen Verfahrens bei Verwendung eines Lösungsmittelgemisches von Ethanol und Wasser in einem Volumenverhältnis von 1 : 1 bei einer Durchmischung mit einer Rührerdrehzahl von 500 U/min und einem Leistungseintrag von 11 W/L und bei Verwendung eines Farnesen-Isomerengemisches als Einsatzstoff,

Fig. B4 zeigt den Reaktionsverlauf des erfindungsgemäßen Verfahrens bei Verwendung eines Lösungsmittelgemisches von Ethanol und Wasser in einem Volumenverhältnis von 1 : 1, jeweils gerechnet als Reinstoff bei einer Durchmischung mit einer Rührerdrehzahl von 20000 U/min und einem Leistungseintrag von 662 W/L und bei Verwendung eines Farnesen-Isomerengemisches als Einsatzstoff,

Fig. C1 zeigt den Reaktionsverlauf eines Verfahrens bei Verwendung von Wasser als Lösungsmittel bei einer Durchmischung mit einer Rührerdrehzahl von 500 U/min und einem Leistungseintrag von 11 W/L.

In der Figur A1 wird der Reaktionsverlauf eines Verfahrens nach dem Stand der Technik bei Verwendung eines Lösungsmittelgemisches von Methanol und Wasser in einem Volumenverhältnis von 3 : 1, gerechnet als Volumenverhältnis der Reinstoffe, bei einer Durchmischung mit einer Rührerdrehzahl von 500 U/min und einem Leistungseintrag von 11 W/L gezeigt. Auf der x-Achse ist die Reaktionszeit in Minuten aufgetragen. Auf der y-Achse ist die jeweilige Zusammensetzung der Reaktionsmischung, ausgedrückt als Flächen-% einer Gaschromatographie-Analyse, mit punktuellen Einzelmessungen über den Reaktionsverlauf dargestellt. Angegeben ist Farnesen mit einem Dreiecksymbol, das Zielprodukt Methyl (4E,8E)-2-acetyl-5,9,13-trimethyl-tetradeca-4,8,12-trienoat (und Isomere hiervon), auch bezeichnet als **X,** mit einem Quadratsymbol, Farnesylaceton **Y** (und Isomere hiervon) mit einem Kreissymbol und die Produktsumme mit einem Sternsymbol.

In der Figur A2 wird der Reaktionsverlauf eines Verfahrens nach dem Stand der Technik bei Verwendung eines Lösungsmittelgemisches von Methanol und Wasser in einem Volumenverhältnis von 1 : 3, gerechnet als Volumenverhältnis der Reinstoffe, bei einer Durchmischung mit einer Rührerdrehzahl von 500 U/min und einem Leistungseintrag von 11 W/L gezeigt. Auf der x-Achse ist die Reaktionszeit in Minuten aufgetragen. Auf der y-Achse ist die jeweilige Zusammensetzung der Reaktionsmischung, ausgedrückt als Flächen-% einer Gaschromatographie-Analyse, mit punktuellen Einzelmessungen über den Reaktionsverlauf dargestellt.

Angegeben ist Farnesen mit einem Dreiecksymbol, das Zielprodukt Methyl (4E,8E)-2-acetyl-5,9,13-trimethyltetradeca-4,8,12-trienoat (und Isomere hiervon), auch bezeichnet als **X,** mit einem Quadratsymbol, Farnesylaceton **Y** (und Isomere hiervon) mit einem Kreissymbol und die Produktsumme mit einem Sternsymbol.

In der Figur A3 wird der Reaktionsverlauf eines Verfahrens nach dem Stand der Technik bei Verwendung eines Lösungsmittelgemisches von Methanol und Wasser in einem Volumenverhältnis von 1 : 3, gerechnet als Volumenverhältnis der Reinstoffe, bei einer Durchmischung mit einer Rührerdrehzahl von 20000 U/min und einem Leistungseintrag von 662 W/L gezeigt.

In der Figur A4 wird der Reaktionsverlauf eines Verfahrens nach dem Stand der Technik bei Verwendung eines Lösungsmittelgemisches von Methanol und Wasser in einem Volumenverhältnis von 1 : 1, gerechnet als Volumenverhältnis der Reinstoffe, bei einer Durchmischung mit einer Rührerdrehzahl von 20000 U/min und einem Leistungseintrag von 662 W/L gezeigt. Auf der x-Achse ist die Reaktionszeit in Minuten aufgetragen. Auf der y-Achse ist die jeweilige Zusammensetzung der Reaktionsmischung, ausgedrückt als Flächen-% einer Gaschromatographie-Analyse, mit punktuellen Einzelmessungen über den Reaktionsverlauf dargestellt.

Angegeben ist Farnesen mit einem Dreiecksymbol, das Zielprodukt Methyl (4E,8E)-2-acetyl-5,9,13-trimethyltetradeca-4,8,12-trienoat (und Isomere hiervon), auch bezeichnet als **X,** mit einem Quadratsymbol, Farnesylaceton **Y** (und Isomere hiervon) mit einem Kreissymbol und die Produktsumme mit einem Sternsymbol.

Die Figuren A1 und A2 zeigen Zeit/Umsatz-Kurven nach dem Stand der Technik. Die Reaktionsgeschwindigkeit wird nicht signifikant bei schneller Durchmischung (hoher Leistungseintrag) erhöht, wie der Vergleich der Beispiele A2 und A3 zeigt. Auch bei einem hohen Leistungseintrag wird nach 6 Stunden Reaktionszeit weniger als 40%-Umsatz zum Zielprodukt erreicht.

Überraschenderweise wurde gefunden, dass die Reaktionsgeschwindigkeit sich signifikant erhöht (Beispiel B2) im Vergleich zum Stand der Technik (Beispiel A4), wenn ein spezielles Lösungsmittel/Wasser-Verhältnis (hier

Ethanol: Wasser = 1 : 1) eingestellt wird und dann mit einem hohen Leistungseintrag durchmischt wird.

In der Figur B1 wird der Reaktionsverlauf des erfindungsgemäßen Verfahrens bei Verwendung eines Lösungsmittelgemisches von Ethanol und Wasser in einem Volumenverhältnis von 1 : 1 bei einer Durchmischung mit einer Rührerdrehzahl von 500 U/min und einem Leistungseintrag von 11 W/L gezeigt. Auf der x-Achse ist die Reaktionszeit in Minuten aufgetragen. Auf der y-Achse ist die jeweilige Zusammensetzung der Reaktionsmischung, ausgedrückt als Flächen-% einer Gaschromatographie-Analyse, mit punktuellen Einzelmessungen über den Reaktionsverlauf dargestellt.

Angegeben ist Farnesen mit einem Dreiecksymbol, das Zielprodukt Methyl (4E,8E)-2-acetyl-5,9,13-trimethyltetradeca-4,8,12-trienoat (und Isomere hiervon), auch bezeichnet als **X,** mit einem Quadratsymbol, Farnesylaceton **Y** (und Isomere hiervon) mit einem Kreissymbol und die Produktsumme mit einem Sternsymbol.

In der Figur B2 wird der Reaktionsverlauf des erfindungsgemäßen Verfahrens bei Verwendung eines Lösungsmittelgemisches von Ethanol und Wasser in einem Volumenverhältnis von 1:1 bei einer Durchmischung mit einer Rührerdrehzahl von 20000 U/min und einem Leistungseintrag von 662 W/L gezeigt. Auf der x-Achse ist die Reaktionszeit in Minuten aufgetragen. Auf der y-Achse ist die jeweilige Zusammensetzung der Reaktionsmischung, ausgedrückt als Flächen-% einer Gaschromatographie-Analyse, mit punktuellen Einzelmessungen über den Reaktionsverlauf dargestellt.

Angegeben ist Farnesen mit einem Dreiecksymbol, das Zielprodukt Methyl (4E,8E)-2-acetyl-5,9,13-trimethyltetradeca-4,8,12-trienoat (und Isomere hiervon), auch bezeichnet als **X,** mit einem Quadratsymbol, Farnesylaceton **Y** (und Isomere hiervon) mit einem Kreissymbol und die Produktsumme mit einem Sternsymbol.

In der Figur B3 wird der Reaktionsverlauf des erfindungsgemäßen Verfahrens bei den gleichen Bedingungen wie der Figur B1, mit der Ausnahme, dass ein Farnesen-Isomerengemisch als Einsatzstoff verwendet wurde, dargestellt. Die Werte, welche sich auf den Umsatz und somit auf die rechtsseitig angeordnete y-Achse beziehen sind das Zielprodukt Methyl (4E,8E)-2-acetyl-5,9,13-trimethyl-tetradeca-4,8,12-trienoat (und Isomere hiervon, Quadratsymbol), auch bezeichnet als **X,** Farnesylaceton **Y** (und Isomere hiervon, Kreissymbol), sowie die Produktsumme (Sternsymbol) umfassend Farnesylaceton **Y** (und Isomeren hiervon) und **X** (und Isomeren hiervon). Das Farnesen mit einem Dreiecksymbol angegeben bezieht sich hingegen auf die linksseitig angeordnete y-Achse.

In der Figur B4 wird der Reaktionsverlauf des erfindungsgemäßen Verfahrens bei den gleichen Bedingungen wie der Figur B2, mit der Ausnahme, dass ein Farnesen-Isomerengemisch als Einsatzstoff verwendet wurde, dargestellt. Die Werte, welche sich auf den Umsatz und somit auf die rechtsseitig angeordnete y-Achse beziehen sind das Zielprodukt Methyl (4E,8E)-2-acetyl-5,9,13-trimethyl-tetradeca-4,8,12-trienoat (und Isomere hiervon, Quadratsymbol), auch bezeichnet als **X,** Farnesylaceton **Y** (und Isomere hiervon, Kreissymbol), sowie die Produktsumme (Sternsymbol) umfassend Farnesylaceton **Y** (und Isomeren hiervon) und **X** (und Isomeren hiervon). Das Farnesen mit einem Dreiecksymbol angegeben bezieht sich hingegen auf die linksseitig angeordnete y-Achse.

In der Figur C1 wird der Reaktionsverlauf eines Verfahrens bei Verwendung von Wasser als Lösungsmittel bei einer Durchmischung mit einer Rührerdrehzahl von 500 U/min und einem Leistungseintrag von 11 W/L gezeigt. Auf der x-Achse ist die Reaktionszeit in Minuten aufgetragen. Auf der y-Achse ist die jeweilige Zusammensetzung der Reaktionsmischung, ausgedrückt als Flächen-% einer Gaschromatographie-Analyse, mit punktuellen Einzelmessungen über den Reaktionsverlauf dargestellt.

Angegeben ist Farnesen mit einem Dreiecksymbol, das Zielprodukt Methyl (4E,8E)-2-acetyl-5,9,13-trimethyltetradeca-4,8,12-trienoat (und Isomere hiervon), auch bezeichnet als **X,** mit einem Quadratsymbol, Farnesylaceton **Y** (und Isomere hiervon) mit einem Kreissymbol und die Produktsumme mit einem Sternsymbol.

Figur C1 zeigt Zeit/Umsatz-Kurven nach dem Stand der Technik mit reinem Wasser als Lösungsmittel. Die Reaktionsgeschwindigkeit ist hier bei gleichem Leistungseintrag vergleichbar mit den Versuchen bei denen Ethanol oder Methanol zugegeben werden (Vergleiche C1 mit A2 und B1).

Überraschenderweise führt also eine spezielle Lösungsmittelkombination bei gleichzeitig stark erhöhten Leistungseinträgen (B 2) zu deutlich erhöhten Reaktionsgeschwindigkeiten, die für ein wirtschaftliches Verfahren notwendig sind.

Die nachfolgenden Beispiele beziehen sich gemäß Ihren Bezeichnungen auf die zugehörigen Figuren. Die für alle Beispiele geltende erfindungsgemäße katalytische Umsetzung von β-E-Farnesen (Bedoukian Res., 90 % β-E-Farnesen, >98 % Summe aller Farnesenisomere) mit Acetessigsäuremethylester ist in dem nachfolgenden Schema 1 dargestellt. Das sich bei der Umsetzung bildende Produktgemisch umfasst Farnesylaceton (und Isomeren hiervon) bezeichnet mit **Y** (und Isomere hiervon) und Methyl (4E,8E)-2-acetyl-5,9,13-trimethyl-tetradeca-4,8,12-trienoat (und Isomeren hiervon), bezeichnet mit **X** (und Isomere hiervon). Die Summe von **X** und **Y** wird in allen Figuren und Beispielen als Summe der Produkte bezeichnet.

Schema 1: Schematische Darstellung des sich aus der katalytische Umsetzung von β-E-Farnesen mit Acetessigsäuremethylester (AME) bildenden Farnesylaceton (und Isomeren hiervon) bezeichnet mit **Y** und Methyl (4E,8E)-2-acetyl-5,9,13-trimethyl-tetradeca-4,8,12-trienoat (und Isomeren hiervon) bezeichnet mit **X**.

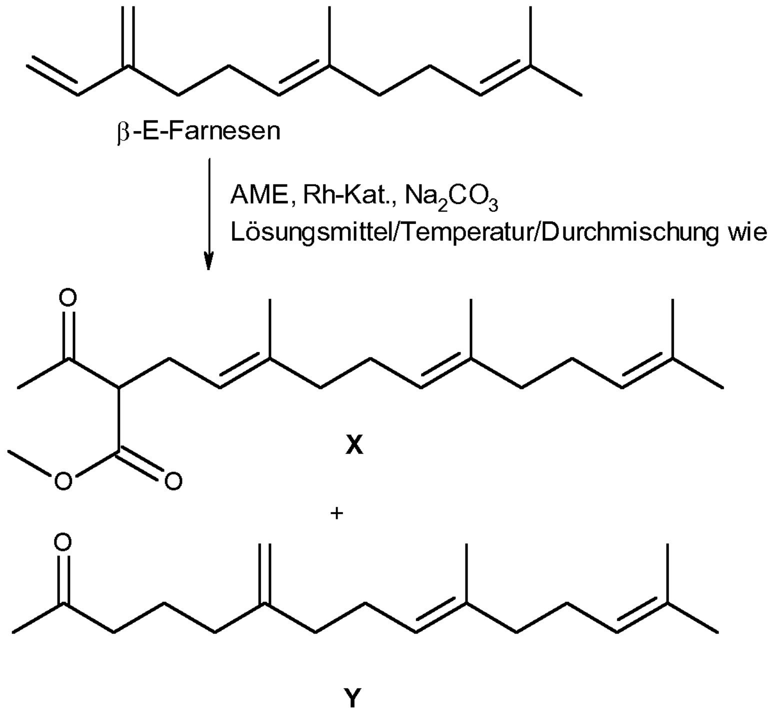


In den Beispielen A1 bis C1 wurde in einer Schutzgasatmosphäre Chlor(1,5-cyclooctadien)rhodium(I) Dimer (0,07 mmol; 35 mg), Trinatriumtri(3-sulfonatophenyl)-phosphin Hydrat (1,1 mmol; 622 mg) und Natriumcarbonat (0,25 mmol; 26,8 mg) in einem 250 mL-Glaskolben vorgelegt. Farnesen (trans-β-Farnesen oder Farnesen-Isomerengemisch entsprechend Tabelle 2, 68,9 mmol; 14,1 g), Acetessigsäuremethylester (170 mmol; 19,7 g) wurde mit Wasser (HPLC-grade) und Ethanol bzw. Methanol in den in Tabelle 2 angegebenen Mengenverhältnissen versetzt. Die eingesetzten Substanzen sind in Tabelle 1 weiter spezifiziert. Die zweiphasige Mischung wurde unter Rühren (500 U/min, KPG-Rührer, Teflon-Rührerblatt halbmondförmig mit 7 cm Durchmesser, oder einem Ultra-Thurrax mit 20000 U/min; Modell IKA T-25 Digital) für 24 Stunden auf 85 °C Innentemperatur erhitzt. Der jeweilige Reaktionsumsatz wurde zu verschiedenen Zeitpunkten per Gaschromatographie-Analyse bestimmt (Probenentnahme aus der dispergierten Mischung und Umsatzbestimmung aus der organischen Phase). Die Gaschromatographie-Parameter waren: Trennsäule: 60 m*0,25 mm HP5, 1.0 μm Filmdicke, Detektor: FID, Trägergas:Wasserstoff, Temp.-Prog.: 80 °C, 10 Min, 20 °C/Min, 250 °C, 30 Min; Bemerkung: von 0,0 bis 9,0 Minuten ist die Integration unterdrückt. $t_R$ (Farnesene) = 18,5-20 min, $t_R$ (AME) = 10,5-12,2 min, $t_R$ (**X**+Isomere) = 29,0 - 32,5 min, $t_R$ (Farnesylaceton **Y**+Isomere) = 23,2 - 25,2 min.

Tabelle 1: Übersicht der in den Beispielen A1 bis D1 eingesetzten Substanzen und Herstellerangaben. K.A. steht für keine Angaben.

| Nr. | Substanz | Molmasse | Reinheit | Hersteller | CAS-Nummer |
|---|---|---|---|---|---|
| 1 | trans-β-Farnesen | 204,35g/mol | k.A. | Bedoukian | 18794-84-8 |
| 2 | Farnesen-Isomerengemisch | 204,35g/mol | k.A. | Sigma-Aldrich | 502-61-4 |
| 3 | Acetessigsäuremethylester (AME) | 116,12g/mol | ≥ 99,0% | Sigma-Aldrich | 105-45-3 |

(fortgesetzt)

| Nr. | Substanz | Molmasse | Reinheit | Hersteller | CAS-Nummer |
|---|---|---|---|---|---|
| 4 | Wasser (HPLC Grade) | 18,01g/mol | ≥ 99,0% | J.T. Baker | 7732-18-5 |
| 5 | Ethanol | 46,07g/mol | ≥ 94,0% | Alfa Aesar | 64-17-5 |
| 6 | Chlor(1,5-cyclooctadiene) rhodium(I) dimer | 493,08g/mol | k.A. | k.A. | 12092-47-6 |
| 7 | Tri(3-sulfonatophenyl) phosphinhydrat, Natriumsalz | 568,40g/mol | ≥ 85,0% | Alfa Aesar | 63995-70-0 |
| 8 | Natriumcarbonat | 105,99g/mol | ≥ 99,8% | Riedel-de Haën | 497-19-8 |

Tabelle 2: Übersicht der in den Beispielen A1 bis C1 eingesetzten Mengenverhältnisse, Rührerdrehzahlen und Leistungseinträge.

| Beispiel | Temp. innen [°C] | U/min | Leistungseintrag [W/L] | Farnesen | Lösungsmittel, Volumina |
|---|---|---|---|---|---|
| A1 | 85 | 500 | 11 | trans-β-Farnesen | Methanol:Wasser = 7,5mL:2,5mL |
| A2 | 85 | 500 | 11 | trans-β-Farnesen | Methanol:Wasser = 2,5mL:7,5mL |
| A3* | 85 | 20000 | 662 | trans-β-Farnesen | Methanol:Wasser = 3,8mL:11,2mL |
| A4* | 85 | 20000 | 662 | trans-β-Farnesen | Methanol:Wasser = 7,5mL:7,5mL |
| B1 | 85 | 500 | 11 | trans-β-Farnesen | Ethanol:Wasser = 5mL: 5mL |
| B2* | 85 | 20000 | 662 | trans-β-Farnesen | Ethanol:Wasser =7,5mL: 7,5mL |
| B3 | 85 | 500 | 11 | Farnesen-Isomerengemisch | Ethanol:Wasser =5mL: 5mL |
| B4* | 85 | 20000 | 662 | Farnesen - Isomerengemisch | Ethanol:Wasser =7,5mL: 7,5mL |
| C1 | 85 | 500 | 11 | trans-β-Farnesen | Wasser (10mL) |
| * Ansatzgröße aller Lösungsmittel, Volumina und Reagenzien wurden um den Faktor 1,5 vergrößert. | | | | | |

Beispiel D1:

Umsetzung von β-E-Farnesen zu Hexahydrofarnesylaceton.

Schema 2: Schematische Darstellung des sich aus der katalytischen Umsetzung von β-E-Farnesen mit Acetessigsäuremethylester (AME) bildenden Hexahydrofarnesylaceton.

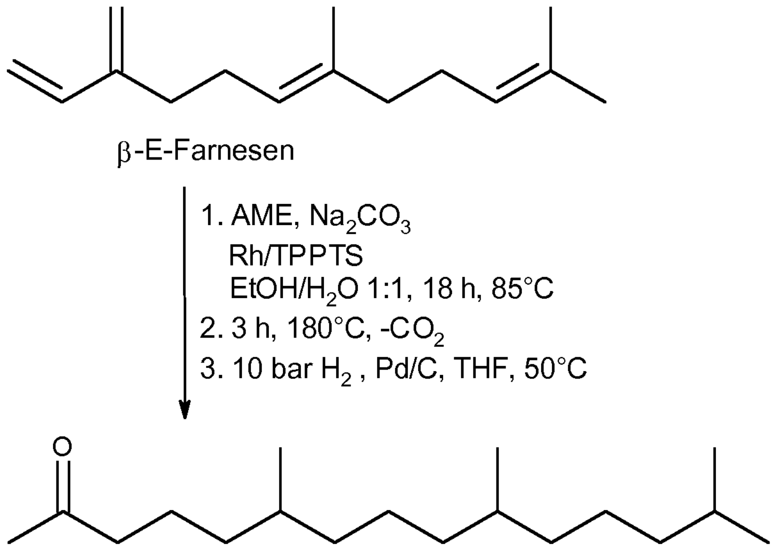


Hexahydrofarneselaceton

Ein Reaktionsaustrag, hergestellt entsprechend Beispiel B2 (Ansatzgröße 16,6 mmol; 3,39 g β-E-Farnesen), wurde auf Raumtemperatur abkühlen gelassen. Vom Austrag (gelb, 2-phasig) wurde die organische Phase isoliert und die wässrige Phase mit Toluol (5 mL) extrahiert. Die organischen Phasen wurden vereinigt und unter Vakuum bei einer Temperatur von 70 °C das Lösungsmittel abgetrennt. Es wurden 4,91 g eines gelben Öls erhalten. Dazu wurde Wasser (HPLC-grade, 10 mL) gegeben und unter Rühren in einem 20 mL-Stahlautoklaven für 3 Stunden auf eine Temperatur von 180 °C erhitzt. Es erfolgte ein Druckanstieg auf 22 bar absolut. Anschließend wurde auf Raumtemperatur gekühlt und entspannt. Der Austrag wurde mit Toluol (2x6 mL) extrahiert und die vereinigten organischen Phasen über Magnesiumsulfat getrocknet und unter Vakuum das Lösungsmittel abgetrennt. Es wurden 3,54 g gelbes Öl erhalten. Das erhaltene Öl wurde mit THF (wasserfrei, 20 mL) und Pd/C (Palladium (10 %) auf Aktivkohle, Alfa Aeser, 350 mg) versetzt. Die Mischung wurde in einen 60 mL-Stahlautoklaven überführt und Wasserstoff (10 bar) aufgepresst. Es wurde unter Rühren für 18 Stunden auf eine Temperatur von 50 °C erhitzt. Danach wurde auf Raumtemperatur abgekühlt und entspannt. Der Reaktionsaustrag (schwarze Suspension) wurde filtriert und unter Vakuum das Lösungsmittel abgetrennt. Erhalten wurden 3,50 g Hexahydrofarnesylaceton als farbloses Öl.

Beispiel E1:

In der nachfolgenden Tabellen 3 und 4 sind die Parameter zur Bestimmung des Leistungseintrages wie zuvor in der Beschreibung ausgeführt, für ein Rotor-Stator-Rührsystem dargestellt.

Tabelle 3: Übersicht der Parameter zur Bestimmung des Leistungseintrages des eingesetzten Rotor-Stator-Rührsystems (Modell: IKA T-25 Digital, Ultra-Thurrax mit 20000 U/min, durchgeführt mit Wasser).

| Masse | Zeit | Temp. | $CpW_{quer}$ | $\Delta H_{quer}$ | Dichte | Vol. | Temperaturanstieg |
|---|---|---|---|---|---|---|---|
| [kg] | [min] | [°C] | [kJ/ (kg*K)] | [kJ/kg] | [kg/m$^3$] | [L] | [°C] |
| 0,56 | 0 | 22 | 4,188 | 2445,8 | 998,4 | 0,561 | |
| | 5 | 26,9 | | | | | 4,9 |
| | 10 | 33,8 | | | | | 6,9 |
| | 15 | 39,3 | | | | | 5,5 |
| | 21 | 46,3 | | | | | 7 |
| | 25 | 51,7 | | | | | 5,4 |
| | 30 | 57,4 | | | | | 5,7 |
| | 35 | 62 | | | | | 4,6 |

(fortgesetzt)

| Masse | Zeit | Temp. | $CpW_{quer}$ | $\Delta$ $H_{quer}$ | Dichte | Vol. | Temperaturanstieg |
|---|---|---|---|---|---|---|---|
| [kg] | [min] | [°C] | [kJ/ (kg*K)] | [kJ/kg] | [kg/m$^3$] | [L] | [°C] |
| | 40 | 67,7 | | | | | 5,7 |
| | 45 | 72,7 | | | | | 5 |
| | 51 | 79,4 | | | | | 6,7 |
| | 55 | 83,2 | | | | | 3,8 |
| | 60 | 87,9 | | | | | 4,7 |
| | 65 | 92,8 | | | | | 4,9 |
| | 70 | 96,5 | | | | | 3,7 |
| 0,548 | 74 | 98,7 | 4,218 | 2267,3 | 947,9 | 0,578 | 2,2 |

Tabelle 4: Übersicht der Parameter zur Bestimmung des Leistungseintrages eines Rotor-Stator-Rührsystems.

| Versuch mit Wasser | | | |
|---|---|---|---|
| mittlere Wärmekapazität von Wasser | $cpW_{quer}$ | 4,203 | kJ/(kg*K) |
| mittlere Verdampfungsenthalpie von Wasser | $\Delta$ $H_{quer}$ | 2356,6 | kJ/kg |
| mittlere Dichte von Wasser | Rho W | 973,2 | kg/m^3 |
| Wärmemenge gesamt | q | 206863 | J |
| eingetragene Leistung | P | 46,6 | W |
| Startvolumen | $V_0$ | 0,56 | I |
| Leistungseintrag bzgl. Startvolumen | $P/V_0$ | 83,2 | W/I |
| Rotordrehzahl | n | 20000 | /min |
| Durchmesser Rotor | $d_R$ | 0,018 | m |
| Leistungsbeiwert Rotor | Ne | 0,68 | |
| Umfangsgeschwindigkeit Rotor | $v_u$ | 18,85 | m/s |
| Übertragung auf Reaktionsgemisch | | | |
| Dichte Reaktionsgemisch | $\rho$ | 940 | kg/m^3 |
| Volumen Reaktionsgemisch | V | 0,068 | I |
| Übertrag Rührleistung Reaktionsgemisch | P | 45 | W |
| Leistungseintrag in Reaktionsgemisch | P/V | 662 | W/I |

Beispiel E2

In der nachfolgenden Tabelle 5 sind die Parameter zur Bestimmung des Leistungseintrages eines Rührersystems bei Verwendung eines Halbmondrührers dargestellt.

Tabelle 5: Übersicht der Parameter zur Bestimmung des Leistungseintrages eines Rührersystems für einen Halbmondrührers.

| Bestimmung des Leistungseintrages bei dem Halbmondrührer | | | |
|---|---|---|---|
| | | | |
| Durchmesser Rührer | $d_R$ | 0,07 | m |

(fortgesetzt)

| Bestimmung des Leistungseintrages bei dem Halbmondrührer | | | | | |
|---|---|---|---|---|---|
| Volumen Reaktionsgemisch | | | V | 0,046 | l |
| Dichte Reaktionsgemisch/ zu rührenden Systems | | | ρ | 940 | $kg/m^3$ |
| Drehzahl | Drehmoment | Rührleistung | Leistungsbeiwert | Leistungseintrag | Umfangsgeschwindigkeit |
| n | M | P | Ne | P/V | v_u |
| [min] | [Ncm] | [W] | [-] | [W/l] | [m/s] |
| 400 | 0,93 | 0,39 | 0,83 | 8,5 | 1,47 |
| 450 | 0,95 | 0,45 | 0,67 | 9,7 | 1,65 |
| 500 | 0,97 | 0,51 | 0,56 | 11 | 1,83 |
| 550 | 0,99 | 0,57 | 0,47 | 12,4 | 2,02 |
| 600 | 1,01 | 0,63 | 0,4 | 13,8 | 2,2 |

**Patentansprüche**

**1.** Verfahren zur Herstellung von Ketoverbindungen der allgemeinen Formel (I)

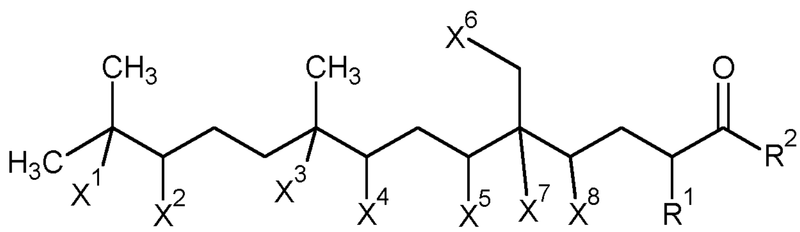

(I)

wobei

$R^1$ für Wasserstoff oder einen Rest $-C(O)OR^3$ steht, wobei
$R^3$ für $C_1$-$C_4$-Alkyl steht;
$R^2$ für $C_1$-$C_4$-Alkyl steht;
$X^1$ und $X^2$ beide für Wasserstoff oder gemeinsam für den Bindungsanteil einer Doppelbindung zwischen den Kohlenstoffatomen, an die sie gebunden sind, stehen;
$X^3$ und $X^4$ beide für Wasserstoff oder gemeinsam für den Bindungsanteil einer Doppelbindung zwischen den Kohlenstoffatomen, an die sie gebunden sind, stehen;
$X^5$, $X^6$, $X^7$ und $X^8$ für Wasserstoff stehen;
wobei eine der Kombinationen der Reste $X^5$ und $X^7$, $X^6$ und $X^7$ oder $X^7$ und $X^8$ auch für den Bindungsanteil einer Doppelbindung zwischen den Kohlenstoffatomen, an die sie gebunden sind, stehen kann,
und Isomere und Mischungen davon,

bei dem man

a) wenigstens eine Farnesen-Verbindung der allgemeinen Formel (II)

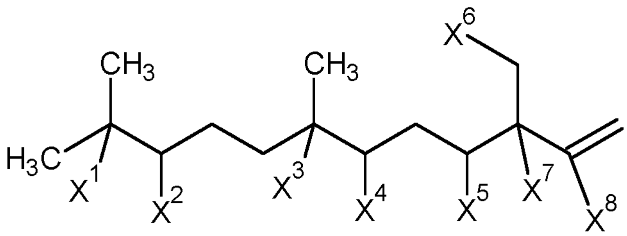

(II)

worin

$X^1$ und $X^2$ gemeinsam für den Bindungsanteil einer Doppelbindung zwischen den Kohlenstoffatomen, an die sie gebunden sind, stehen;
$X^3$ und $X^4$ gemeinsam für den Bindungsanteil einer Doppelbindung zwischen den Kohlenstoffatomen, an die sie gebunden sind, stehen;
$X^5$ und $X^7$ gemeinsam für den Bindungsanteil einer Doppelbindung zwischen den Kohlenstoffatomen, an die sie gebunden sind, stehen unter der Maßgabe, dass $X^6$ für Wasserstoff steht; oder
$X^6$ und $X^7$ gemeinsam für den Bindungsanteil einer Doppelbindung zwischen den Kohlenstoffatomen, an die sie gebunden sind, stehen unter der Maßgabe, dass $X^5$ für Wasserstoff steht, und
$X^8$ für Wasserstoff steht;

mit einem β-Ketoester der allgemeinen Formel (III)

$R^2$-CO-$CH_2$-$R^1$ (III)

worin

$R^1$ für einen Rest -C(O)O$R^3$ steht,
wobei $R^3$ für $C_1$-$C_4$-Alkyl steht;
in Gegenwart eines Katalysators und eines Lösungsmittel/Wasser-Gemischs einer Umsetzung unterwirft, wobei man das Reaktionsgemisch einer Dispergierung mit mindestens einem Mischer bei einer Reynoldszahl von größer als $10^4$ unterzieht,
unter Erhalt einer Verbindung der Formel (I-a),

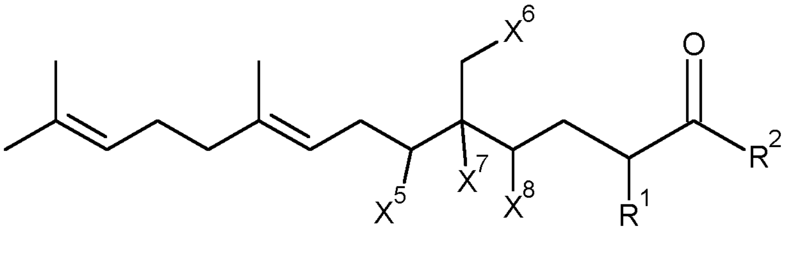

(I-a)

worin

$R^1$ für einen Rest -C(O)O$R^3$ steht, wobei $R^3$ für $C_1$-$C_4$-Alkyl steht;
$R^2$ für $C_1$-$C_4$-Alkyl steht;
eine der Kombinationen der Reste $X^5$ und $X^7$, $X^6$ und $X^7$ oder $X^7$ und $X^8$ gemeinsam für den Bindungsanteil einer Doppelbindung zwischen den Kohlenstoffatomen, an die sie gebunden sind, steht, und die übrigen Reste $X^5$, $X^6$, $X^7$ und $X^8$ für Wasserstoff stehen;

und Isomere und Mischungen davon;
b) gegebenenfalls das in Schritt a) erhaltene Reaktionsgemisch einer Decarboxylierung unterzieht, wobei eine Verbindung der Formel (I-b),

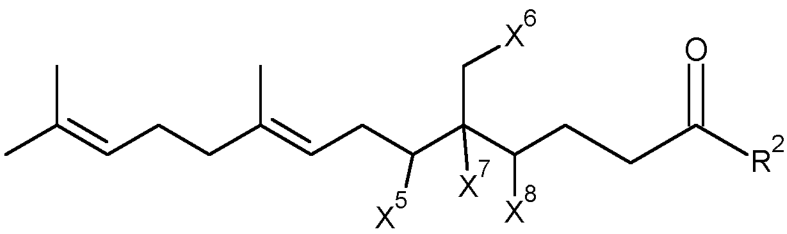

(I-b)

worin

$R^2$ für $C_1$-$C_4$-Alkyl steht; und
eine der Kombinationen der Reste $X^5$ und $X^7$, $X^6$ und $X^7$ oder $X^7$ und $X^8$ gemeinsam für den Bindungsanteil einer Doppelbindung zwischen den Kohlenstoffatomen, an die sie gebunden sind, steht, und die übrigen Reste $X^5$, $X^6$, $X^7$ und $X^8$ für Wasserstoff stehen;
und Isomere und Mischungen davon erhalten wird;

c) gegebenenfalls das in Schritt b) erhaltene Reaktionsgemisch einer Hydrierung unterzieht, wobei eine Verbindung der Formel (I-c)

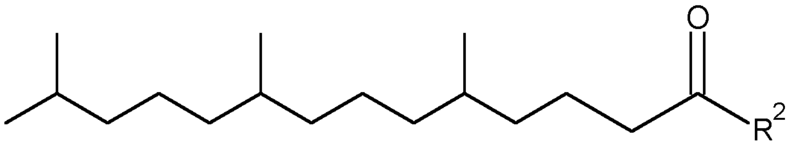

(I-c)

worin

$R^2$ für $C_1$-$C_4$-Alkyl steht;
erhalten wird;
wobei das Lösungsmittel des Lösungsmittel/Wasser-Gemisches in Schritt a) ausgewählt ist unter Ethanol, n-Propanol, iso-Propanol, n-Butanol, t-Amylalkohol, Monoethylenglycol, Diethylenglycol, Tetrahydrofuran, Dioxan, Methyl-tert-butylether, 1,2-Dimethoxyethan, Diethylenglykoldimethylether, Triethylenglykoldimethylether, Acetonitril, Aceton, Essigsäuremethylester, Essigsäureethylester, Dimethylformamid, N-Methylpyrrolidon und Mischungen hiervon.

**2.** Verfahren nach Anspruch 1 zur Herstellung von Farnesylaceton der Formel (I-aA),

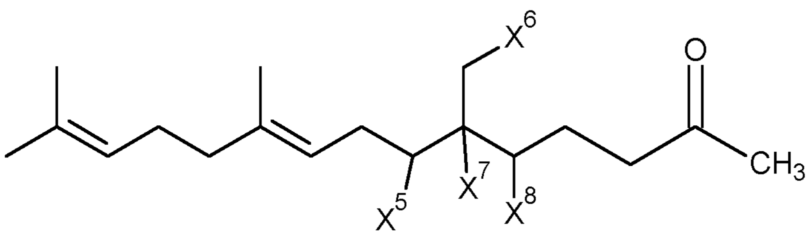

(I-aA)

worin

eine der Kombinationen der Reste $X^5$ und $X^7$, $X^6$ und $X^7$ oder $X^7$ und $X^8$ gemeinsam für den Bindungsanteil einer Doppelbindung zwischen den Kohlenstoffatomen, an die sie gebunden sind, steht, und die übrigen Reste $X^5$, $X^6$, $X^7$ und $X^8$ für Wasserstoff stehen,
und Isomere und Mischungen davon,
wobei das Verfahren die Schritte a) und b) umfasst.

**3.** Verfahren nach Anspruch 1 zur Herstellung von Hexahydrofarnesylaceton (6,10,14-Trimethyl-2-pentadecanon) der Formel (I-bB),

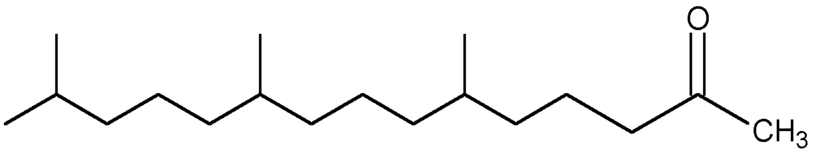

(I-bB)

wobei das Verfahren die Schritte a), b), und c) umfasst.

4. Verfahren nach einem der vorhergehenden Ansprüche, wobei das Lösungsmittel des Lösungsmittel/Wasser-Gemisches in Schritt a) Ethanol oder Diethylenglycol ist.

5. Verfahren nach einem der vorhergehenden Ansprüche, wobei das Lösungsmittel/Wasser-Gemisch in Schritt a) in einem Volumenverhältnis von vorzugsweise 1 : 5 bis 5 : 1 vorliegt.

6. Verfahren nach Anspruch 5, wobei das Lösungsmittel/Wasser-Gemisch in Schritt a) in einem Volumenverhältnis von 1 : 2 bis 2 : 1 vorliegt.

7. Verfahren nach Anspruch 5 oder 6, wobei das Lösungsmittel/Wasser-Gemisch in Schritt a) in einem Volumenverhältnis von 1 : 1 vorliegt.

8. Verfahren nach einem der vorherigen Ansprüche, wobei die Dispergierung in Schritt a) bei einem Leistungseintrag im Bereich von vorzugsweise 0,1 bis 5000 W/L erfolgt.

9. Verfahren nach Anspruch 8, wobei die Dispergierung in Schritt a) bei einem Leistungseintrag im Bereich von vorzugsweise 10 bis 600 W/L erfolgt.

10. Verfahren nach Anspruch 9, wobei die Dispergierung in Schritt a) bei einem Leistungseintrag im Bereich von vorzugsweise 20 bis 100 W/L erfolgt.

11. Verfahren nach einem der vorherigen Ansprüche, wobei die Dispergierung in Schritt a) mit einem Rührer bei einer Umfangsgeschwindigkeit im Bereich von 1 bis 80 m/s erfolgt.

12. Verfahren nach Anspruch 11, wobei die Dispergierung in Schritt a) mit einem Rührer bei einer Umfangsgeschwindigkeit im Bereich von 1,8 bis 30 m/s erfolgt.

13. Verfahren nach einem der vorherigen Ansprüche, wobei die Dispergierung in Schritt a) bei einer Temperatur im Bereich von 50 bis 200 °C erfolgt.

14. Verfahren nach Anspruch 13, wobei die Dispergierung in Schritt a) bei einer Temperatur im Bereich von 60 bis 150 °C erfolgt.

15. Verfahren nach einem der vorherigen Ansprüche, bei dem man das erhaltene Farnesylaceton zur Herstellung von Vitamin E, Isophytol, Dehydro-Isophytol, Hexahydrofarnesylaceton, Tetrahydrofarnesylaceton verwendet.

## Claims

1. A process for preparing keto compounds of the general formula (I)

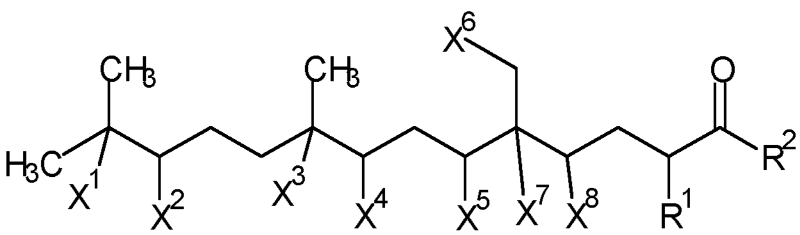

(I)

where

$R^1$ is hydrogen or a $-C(O)OR^3$ radical, where
$R^3$ is $C_1$-$C_4$-alkyl;
$R^2$ is $C_1$-$C_4$-alkyl;
$X^1$ and $X^2$ are both hydrogen or together are the second bond of a double bond between the carbon atoms to which they are bound;
$X^3$ and $X^4$ are both hydrogen or together are the second bond of a double bond between the carbon atoms to which they are bound;
$X^5$, $X^6$, $X^7$ and $X^8$ are each hydrogen;
where one of the combinations of the radicals $X^5$ and $X^7$, $X^6$ and $X^7$ or $X^7$ and $X^8$ can also be the second bond of a double bond between the carbon atoms to which they are bound,
and isomers and mixtures thereof,

where

a) at least one farnesene compound of the general formula (II)

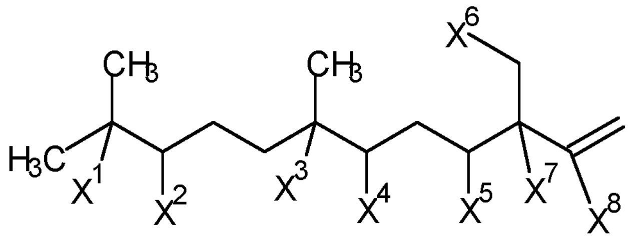

(II)

where

$X^1$ and $X^2$ together are the second bond of a double bond between the carbon atoms to which they are bound;
$X^3$ and $X^4$ together are the second bond of a double bond between the carbon atoms to which they are bound;
$X^5$ and $X^7$ together are the second bond of a double bond between the carbon atoms to which they are bound, with the proviso that $X^6$ is hydrogen; or
$X^6$ and $X^7$ together are the second bond of a double bond between the carbon atoms to which they are bound, with the proviso that $X^5$ is hydrogen, and
$X^8$ is hydrogen;

is subjected to a reaction with a β-keto ester of the general formula (III)

$$R^2\text{-CO-CH}_2\text{-}R^1 \qquad \text{(III)}$$

where

$R^1$ is a $-C(O)OR^3$ radical,
where $R^3$ is $C_1$-$C_4$-alkyl
in the presence of a catalyst and a solvent/water mixture, where the reaction mixture is subjected to dispersing using at least one mixer at a Reynolds number of greater than $10^4$,
to give a compound of the formula (I-a),

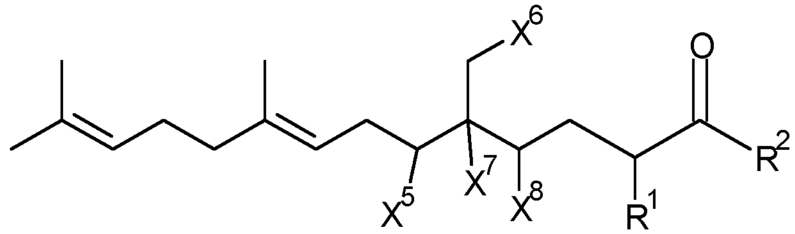

(I-a)

where

$R^1$ is a -C(O)OR$^3$ radical, where $R^3$ is $C_1$-$C_4$-alkyl;
$R^2$ is $C_1$-$C_4$-alkyl;
one of the combinations of the radicals $X^5$ and $X^7$, $X^6$ and $X^7$ or $X^7$ and $X^8$ together is the second bond of a double bond between the carbon atoms to which they are bound and the remaining radicals $X^5$, $X^6$, $X^7$ and $X^8$ are each hydrogen;

and isomers and mixtures thereof;
b) the reaction mixture obtained in step a) is optionally subjected to a decarboxylation to give a compound of the formula (I-b),

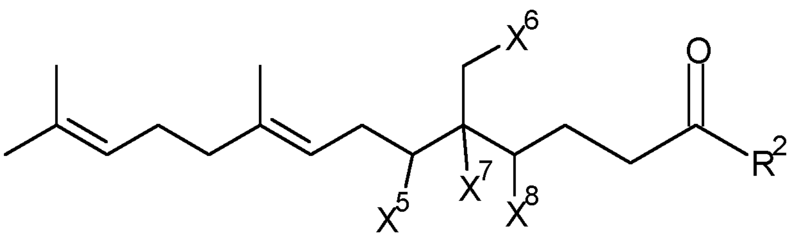
(I-b)

where

$R^2$ is $C_1$-$C_4$-alkyl; and
one of the combinations of the radicals $X^5$ and $X^7$, $X^6$ and $X^7$ or $X^7$ and $X^8$ together is the second bond of a double bond between the carbon atoms to which they are bound and the remaining radicals $X^5$, $X^6$, $X^7$ and $X^8$ are each hydrogen;
and isomers and mixtures thereof;

c) the reaction mixture obtained in step b) is optionally subjected to a hydrogenation to give a compound of the formula (I-c)

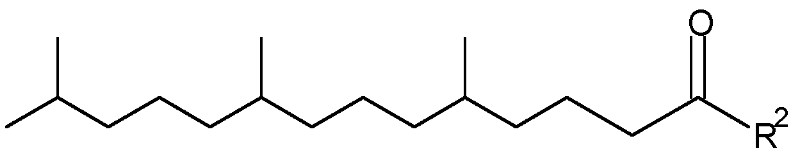
(I-c)

where

$R^2$ is $C_1$-$C_4$-alkyl;
where the solvent of the solvent/water mixture in step a) is selected from among ethanol, n-propanol, isopropanol, n-butanol, t-amyl alcohol, monoethylene glycol, diethylene glycol, tetrahydrofuran, dioxane, methyl tert-butyl ether, 1,2-dimethoxyethane, diethylene glycol dimethyl ether, triethylene glycol dimethyl ether, acetonitrile, acetone, methyl acetate, ethyl acetate, dimethylformamide, N-methylpyrrolidone and mixtures thereof.

**2.** The process according to claim 1 for preparing farnesyl acetone of the formula (I-aA),

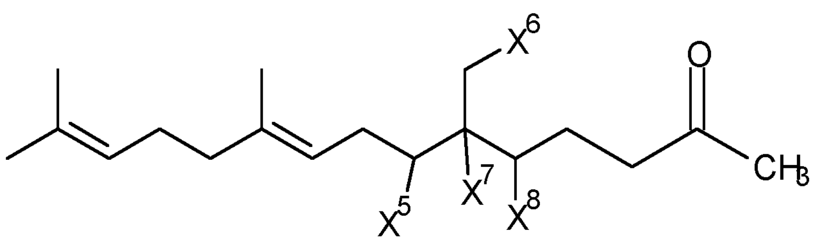
(I-aA)

where

one of the combinations of the radicals $X^5$ and $X^7$, $X^6$ and $X^7$ or $X^7$ and $X^8$ together is the second bond of a double bond between the carbon atoms to which they are bound and the remaining radicals $X^5$, $X^6$, $X^7$ and $X^8$ are each hydrogen,
and isomers and mixtures thereof,
wherein the process comprises the steps a) and b).

3. The process according to claim 1 for preparing hexahydrofarnesyl acetone (6,10,14-trimethyl-2-pentadecanone) of the formula (I-bB),

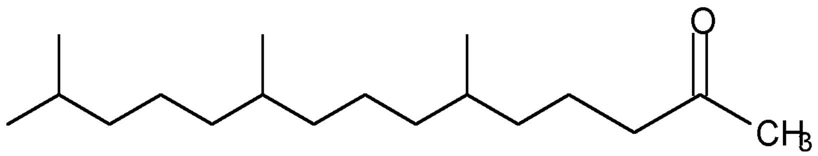

(I-bB)

wherein the process comprises the steps a), b), and c).

4. The process according to any of the preceding claims, wherein the solvent of the solvent/water mixture in step a) is ethanol or diethylene glycol.

5. The process according to any of the preceding claims, wherein the solvent/water mixture in step a) is present in a volume ratio of preferably from 1:5 to 5:1.

6. The process according to claim 5, wherein the solvent/water mixture in step a) is present in a volume ratio of from 1:2 to 2:1.

7. The process according to claim 5 or 6, wherein the solvent/water mixture in step a) is present in a volume ratio of 1:1.

8. The process according to any of the preceding claims, wherein dispersing in step a) is carried out at a power input in the range from preferably 0.1 to 5000 W/l.

9. The process according to claim 8, wherein dispersing in step a) is carried out at a power input in the range from preferably 10 to 600 W/l.

10. The process according to claim 9, wherein dispersing in step a) is carried out at a power input in the range from preferably 20 to 100 W/l.

11. The process according to any of the preceding claims, wherein dispersing in step a) is carried out using a stirrer at a circumferential velocity in the range from 1 to 80 m/s.

12. The process according to claim 11, wherein dispersing in step a) is carried out using a stirrer at a circumferential velocity in the range from 1.8 to 30 m/s.

13. The process according to any of the preceding claims, wherein dispersing in step a) is carried out at a temperature in the range from 50 to 200°C.

14. The process according to claim 13, wherein dispersing in step a) is carried out at a temperature in the range from 60 to 150°C.

15. The process according to any of the preceding claims, in which the farnesyl acetone obtained is used for preparing vitamin E, isophytol, dehydroisophytol, hexahydrofarnesyl acetone, tetrahydrofarnesyl acetone.

**Revendications**

1. Procédé de fabrication de composés céto de la formule générale (I) :

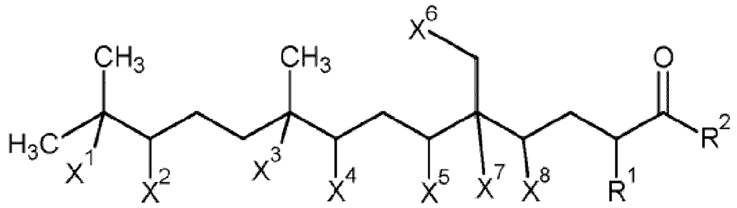

(I)

dans laquelle

$R^1$ représente l'hydrogène ou un radical -C(O)OR$^3$,
$R^3$ représentant un alkyle en $C_1$-$C_4$ ;
$R^2$ représente un alkyle en $C_1$-$C_4$ ;
$X^1$ et $X^2$ représentent tous les deux l'hydrogène ou ensemble la partie de liaison d'une double liaison entre les atomes de carbone auxquels ils sont reliés ; $X^3$ et $X^4$ représentent tous les deux l'hydrogène ou ensemble la partie de liaison d'une double liaison entre les atomes de carbone auxquels ils sont reliés ; $X^5$, $X^6$, $X^7$ et $X^8$ représentent l'hydrogène ;
une des combinaisons des radicaux $X^5$ et $X^7$, $X^6$ et $X^7$ ou $X^7$ et $X^8$ pouvant également représenter la partie de liaison d'une double liaison entre les atomes de carbone auxquels ils sont reliés,
et d'isomères et de mélanges de ceux-ci,

selon lequel

a) au moins un composé de farnésène de la formule générale (II) :

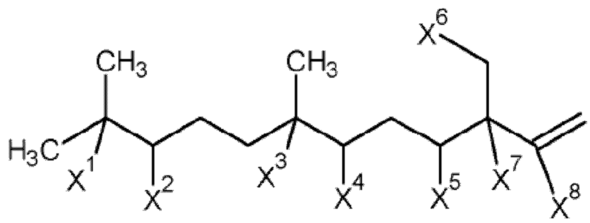

(II)

dans laquelle

$X^1$ et $X^2$ représentent ensemble la partie de liaison d'une double liaison entre les atomes de carbone auxquels ils sont reliés ;
$X^3$ et $X^4$ représentent ensemble la partie de liaison d'une double liaison entre les atomes de carbone auxquels ils sont reliés ;
$X^5$ et $X^7$ représentent ensemble la partie de liaison d'une double liaison entre les atomes de carbone auxquels ils sont reliés, à condition que $X^6$ représente l'hydrogène ; ou
$X^6$ et $X^7$ représentent ensemble la partie de liaison d'une double liaison entre les atomes de carbone auxquels ils sont reliés, à condition que $X^5$ représente l'hydrogène, et
$X^8$ représente l'hydrogène ;

est soumis à une réaction avec un β-cétoester de la formule générale (III) :

$$R^2\text{-CO-CH}_2\text{-}R^1 \qquad \text{(III)}$$

dans laquelle

$R^1$ représente un radical -C(O)OR$^3$, $R^3$ représentant un alkyle en $C_1$-$C_4$,
en présence d'un catalyseur et d'un mélange solvant/eau, le mélange réactionnel étant soumis à une dispersion avec au moins un mélangeur à un nombre de Reynold supérieur à $10^4$,
pour obtenir un composé de la formule (I-a) :

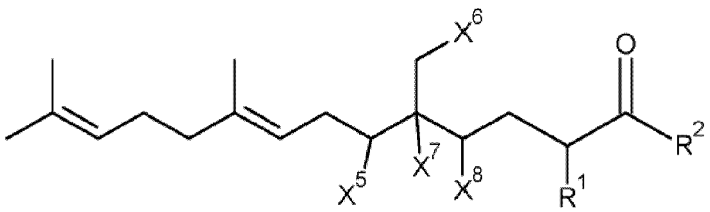

(I-a)

dans laquelle

$R^1$ représente un radical -C(O)O$R^3$,
$R^3$ représentant un alkyle en $C_1$-$C_4$ ;
$R^2$ représente un alkyle en $C_1$-$C_4$ ;
une des combinaisons des radicaux $X^5$ et $X^7$, $X^6$ et $X^7$ ou $X^7$ et $X^8$ représente ensemble la partie de liaison d'une double liaison entre les atomes de carbone auxquels ils sont reliés, et les radicaux $X^5$, $X^6$, $X^7$ et $X^8$ restants représentent l'hydrogène ;

et des isomères et mélanges de celui-ci,
b) le mélange réactionnel obtenu à l'étape a) est éventuellement soumis à une décarboxylation, un composé de la formule (I-b) :

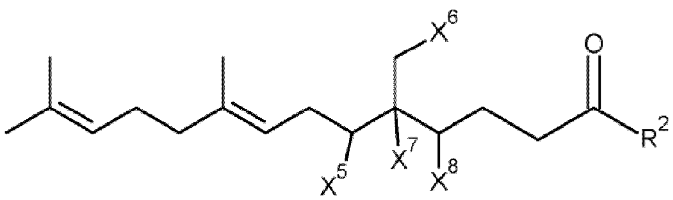

(I-b)

dans laquelle

$R^2$ représente un alkyle en $C_1$-$C_4$ ; et
une des combinaisons des radicaux $X^5$ et $X^7$, $X^6$ et $X^7$ ou $X^7$ et $X^8$ représente ensemble la partie de liaison d'une double liaison entre les atomes de carbone auxquels ils sont reliés, et les radicaux $X^5$, $X^6$, $X^7$ et $X^8$ restants représentent l'hydrogène ;
et des isomères et mélanges de celui-ci étant obtenus ;

c) le mélange réactionnel obtenu à l'étape b) est éventuellement soumis à une hydrogénation, un composé de la formule (I-c) :

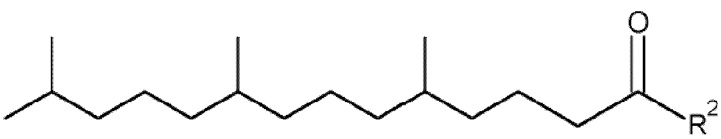

(I-c)

dans laquelle

$R^2$ représente un alkyle en $C_1$-$C_4$ ;
étant obtenu ;
le solvant du mélange solvant/eau à l'étape a) étant choisi parmi l'éthanol, le n-propanol, l'isopropanol, le n-butanol, l'alcool t-amylique, le monoéthylène glycol, le diéthylène glycol, le tétrahydrofurane, le dioxane, l'éther méthyl-tert-butylique, le 1,2-diméthoxyéthane, l'éther diméthylique de diéthylène glycol, l'éther diméthylique de triéthylène glycol, l'acétonitrile, l'acétone, l'ester méthylique de l'acide acétique, l'ester éthylique de l'acide acétique, le diméthylformamide, la N-méthylpyrrolidone et les mélanges de ceux-ci.

**2.** Procédé selon la revendication 1 pour la fabrication de farnésylacétone de la formule (I-aA) :

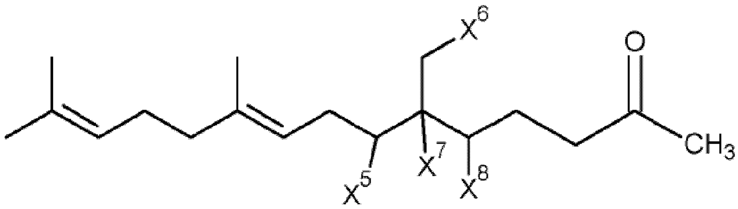

(I-aA)

dans laquelle

une des combinaisons des radicaux $X^5$ et $X^7$, $X^6$ et $X^7$ ou $X^7$ et $X^8$ représente ensemble la partie de liaison d'une double liaison entre les atomes de carbone auxquels ils sont reliés, et les radicaux $X^5$, $X^6$, $X^7$ et $X^8$ restants représentent l'hydrogène ;
et d'isomères et de mélanges de celle-ci,
le procédé comprenant les étapes a) et b).

3. Procédé selon la revendication 1 pour la fabrication d'hexahydrofarnésylacétone (6,10,14-triméthyl-2-pentadécanone) de la formule (I-bB) :

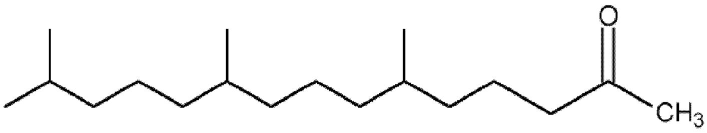

(I-bB)

le procédé comprenant les étapes a), b) et c).

4. Procédé selon l'une quelconque des revendications précédentes, dans lequel le solvant du mélange solvant/eau à l'étape a) est l'éthanol ou le diéthylène glycol.

5. Procédé selon l'une quelconque des revendications précédentes, dans lequel le mélange solvant/eau à l'étape a) présente un rapport de volume de préférence de 1:5 à 5:1.

6. Procédé selon la revendication 5, dans lequel le mélange solvant/eau à l'étape a) présente un rapport de volume de 1:2 à 2:1.

7. Procédé selon la revendication 5 ou 6, dans lequel le mélange solvant/eau à l'étape a) présente un rapport de volume de 1:1.

8. Procédé selon l'une quelconque des revendications précédentes, dans lequel la dispersion à l'étape a) a lieu avec un apport de puissance dans la plage allant de préférence de 0,1 à 5 000 W/l.

9. Procédé selon la revendication 8, dans lequel la dispersion à l'étape a) a lieu avec un apport de puissance dans la plage allant de préférence de 10 à 600 W/l.

10. Procédé selon la revendication 9, dans lequel la dispersion à l'étape a) a lieu avec un apport de puissance dans la plage allant de préférence de 20 à 100 W/l.

11. Procédé selon l'une quelconque des revendications précédentes, dans lequel la dispersion à l'étape a) a lieu avec un agitateur à une vitesse périphérique dans la plage allant de 1 à 80 m/s.

12. Procédé selon la revendication 11, dans lequel la dispersion à l'étape a) a lieu avec un agitateur à une vitesse périphérique dans la plage allant de 1,8 à 30 m/s.

13. Procédé selon l'une quelconque des revendications précédentes, dans lequel la dispersion à l'étape a) a lieu à une température dans la plage allant de 50 à 200 °C.

14. Procédé selon la revendication 13, dans lequel la dispersion à l'étape a) a lieu à une température dans la plage

allant de 60 à 150 °C.

15. Procédé selon l'une quelconque des revendications précédentes, dans lequel la farnésylacétone obtenue est utilisée pour la fabrication de vitamine E, d'isophytol, de déshydro-isophytol, d'hexahydrofarnésylacétone, de tétrahydro-farnésylacétone.

Fig. A1:

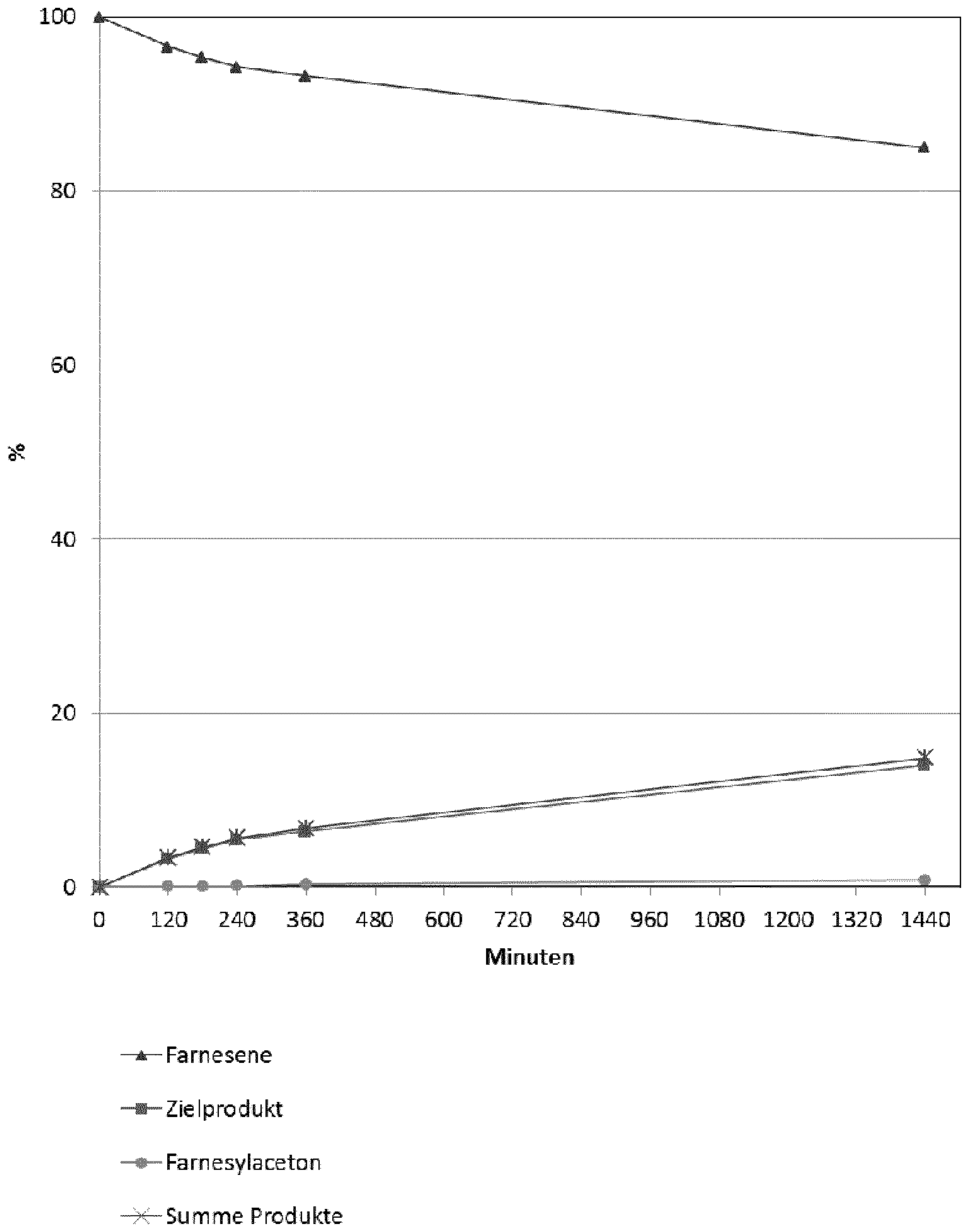
100
80
60
%
40
20
0
0
120
240
360
480
600
720
840
960
1080
1200
1320
1440
Minuten
Farnesene
Zielprodukt
Farnesylaceton
Summe Produkte

Fig. A2:

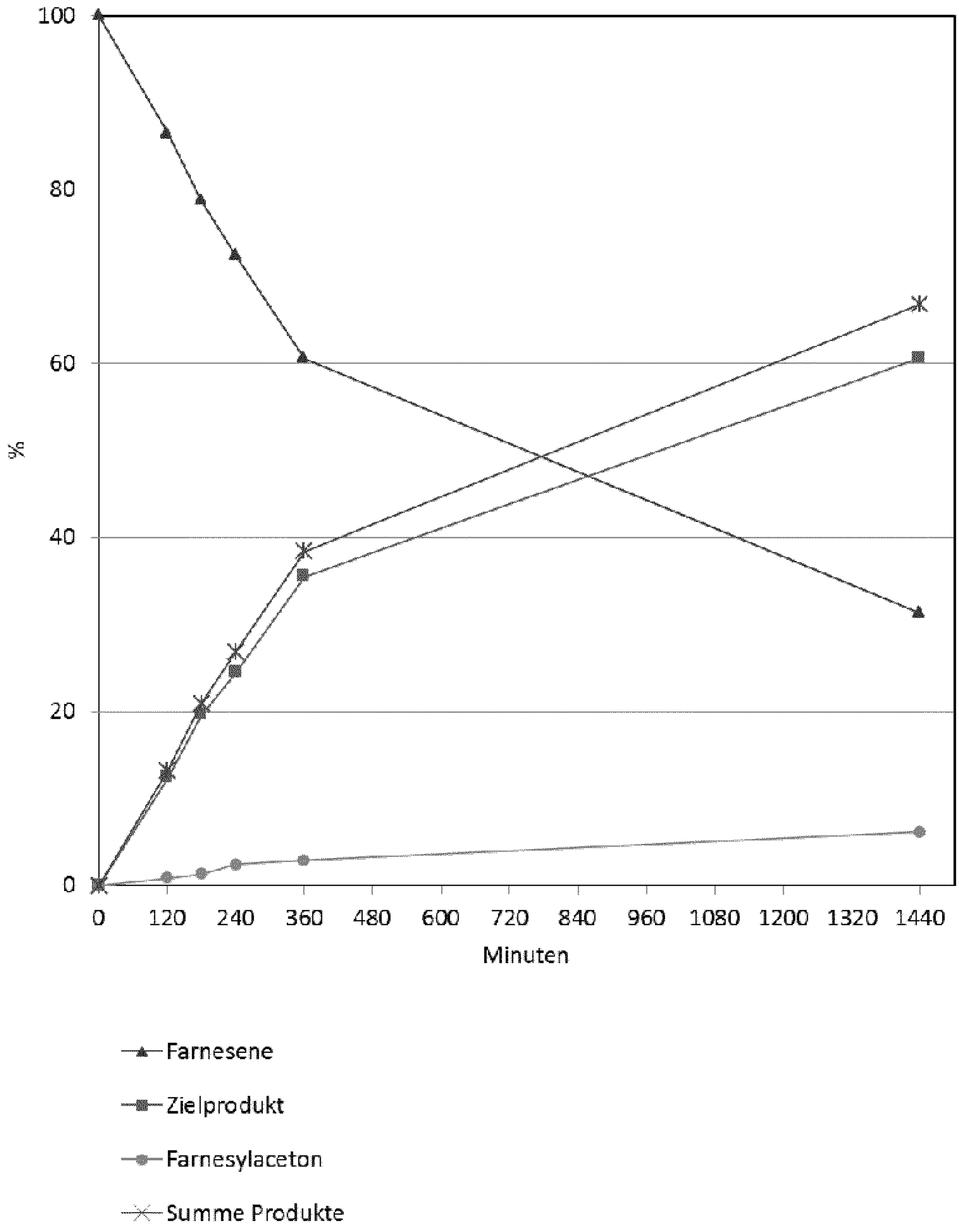
100
80
60
40
20
0
%
0
120
240
360
480
600
720
840
960
1080
1200
1320
1440
Minuten
Farnesene
Zielprodukt
Farnesylaceton
Summe Produkte

Fig. A3:

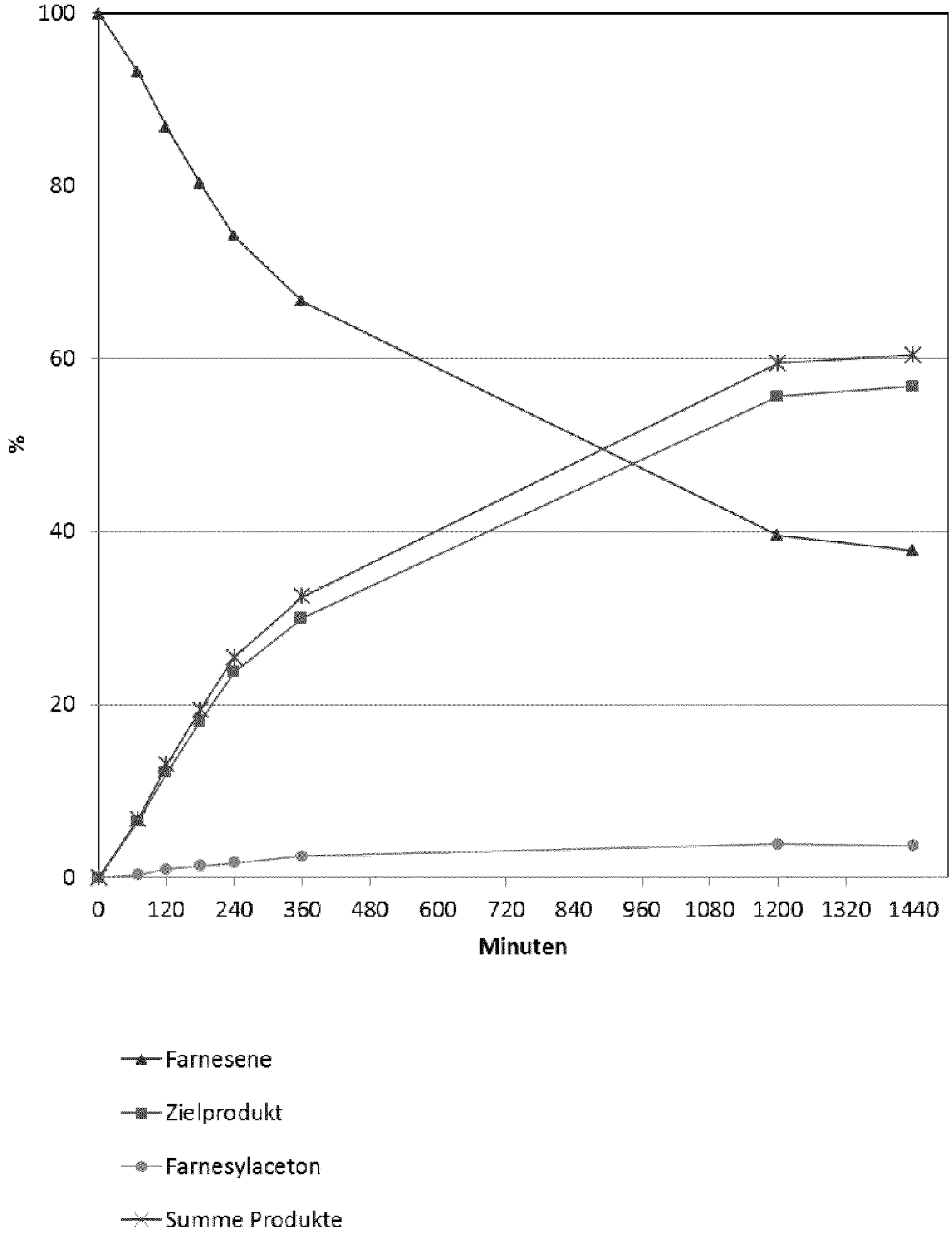
100
80
60
40
20
0
%
0
120
240
360
480
600
720
840
960
1080
1200
1320
1440
Minuten
Farnesene
Zielprodukt
Farnesylaceton
Summe Produkte

Fig. A4:

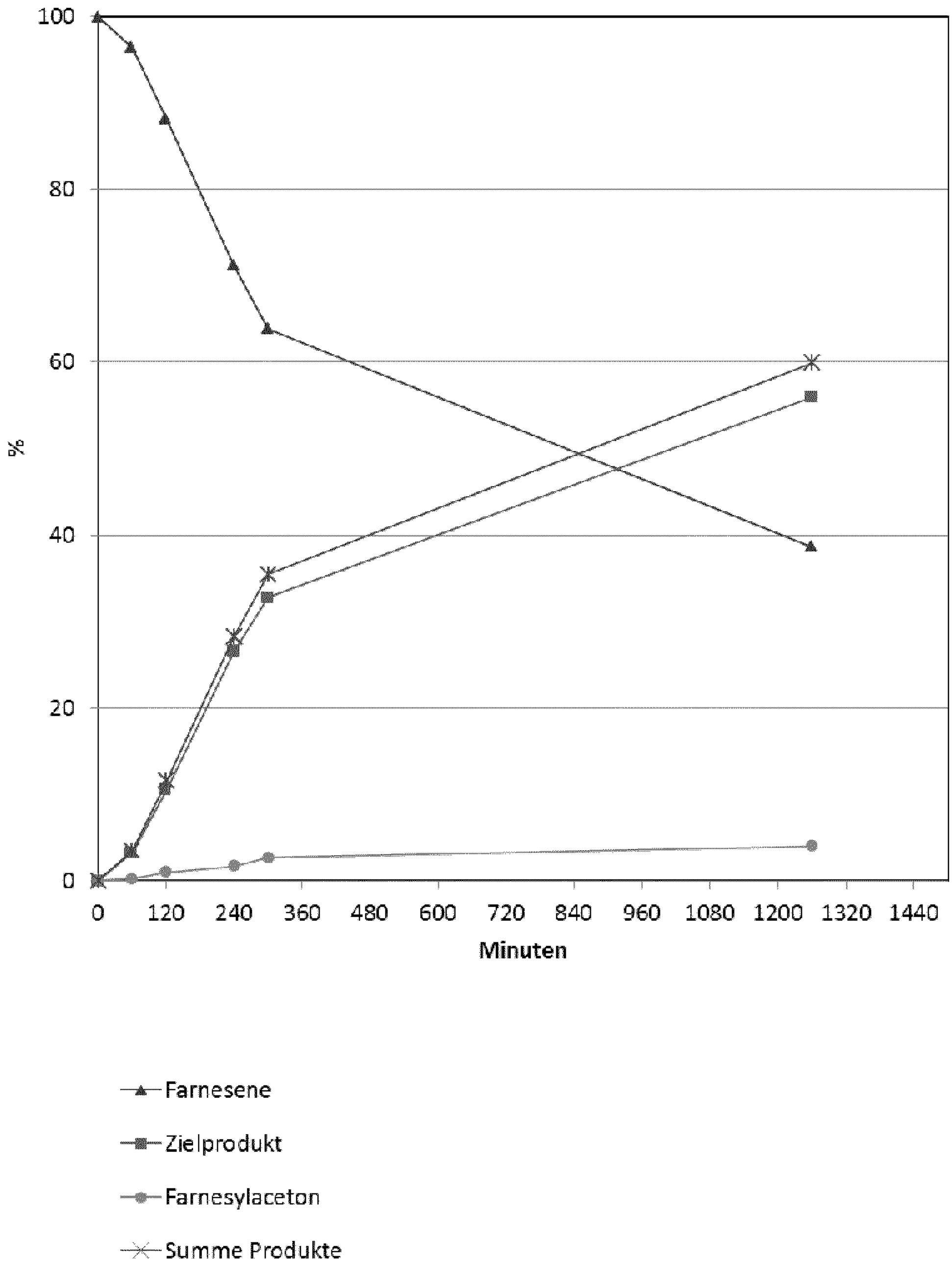
100
80
60
40
20
0
%
0
120
240
360
480
600
720
840
960
1080
1200
1320
1440
Minuten
Farnesene
Zielprodukt
Farnesylaceton
Summe Produkte

Fig. B1:

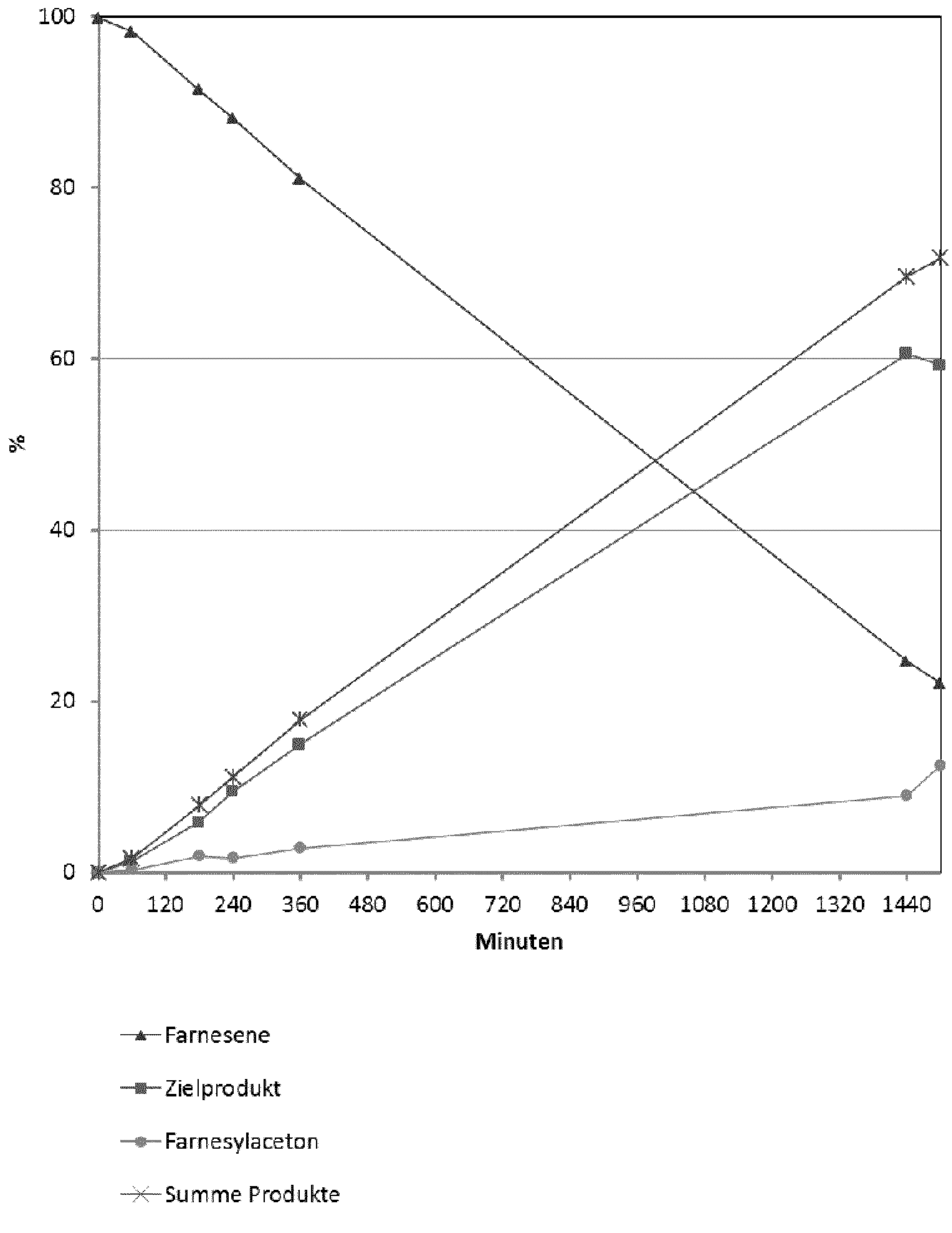
100
80
60
%
40
20
0
0
120
240
360
480
600
720
840
960
1080
1200
1320
1440
Minuten
Farnesene
Zielprodukt
Farnesylaceton
Summe Produkte

Fig. B2:

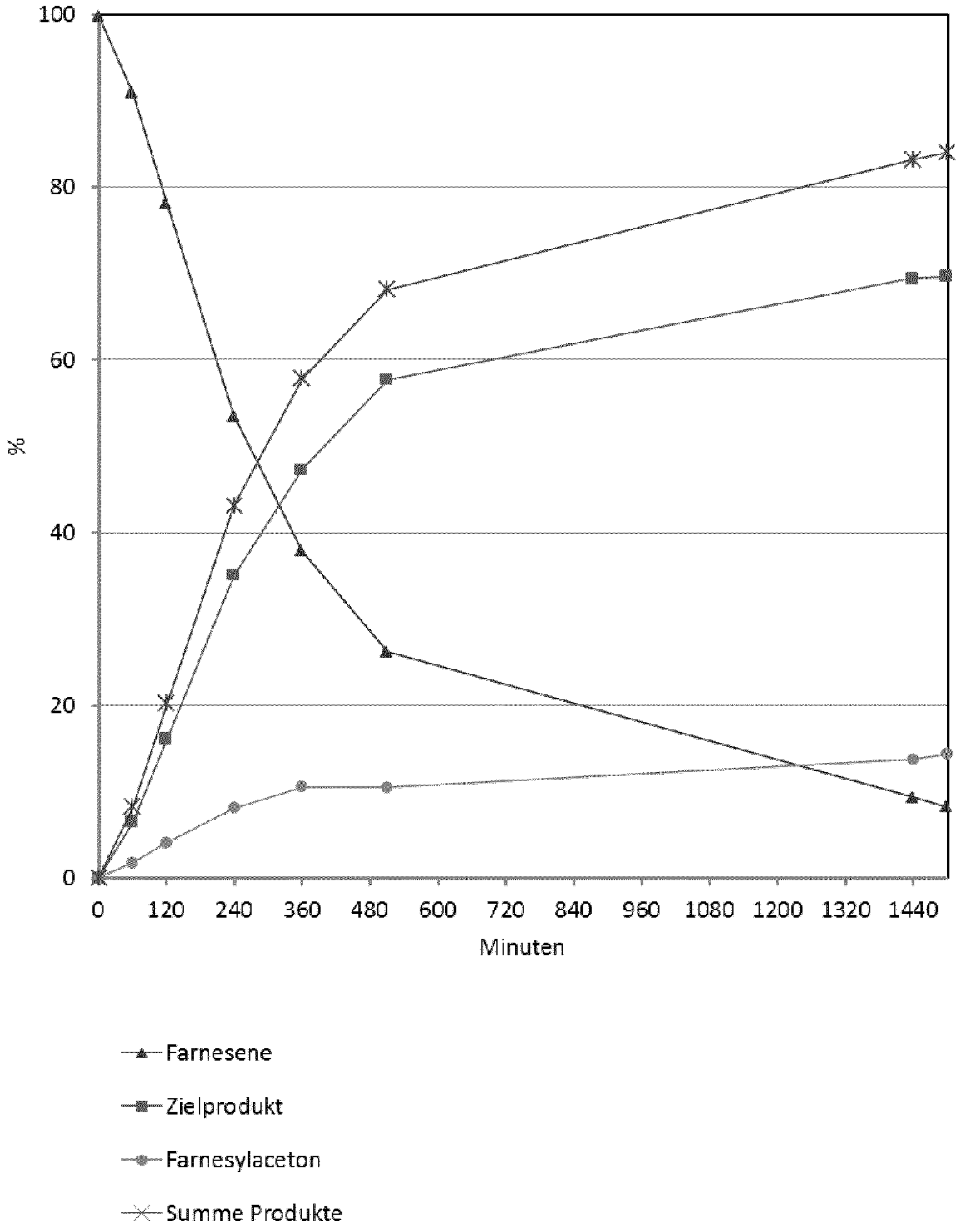
100
80
60
40
20
0
%
0
120
240
360
480
600
720
840
960
1080
1200
1320
1440
Minuten
Farnesene
Zielprodukt
Farnesylaceton
Summe Produkte

Fig. B3:

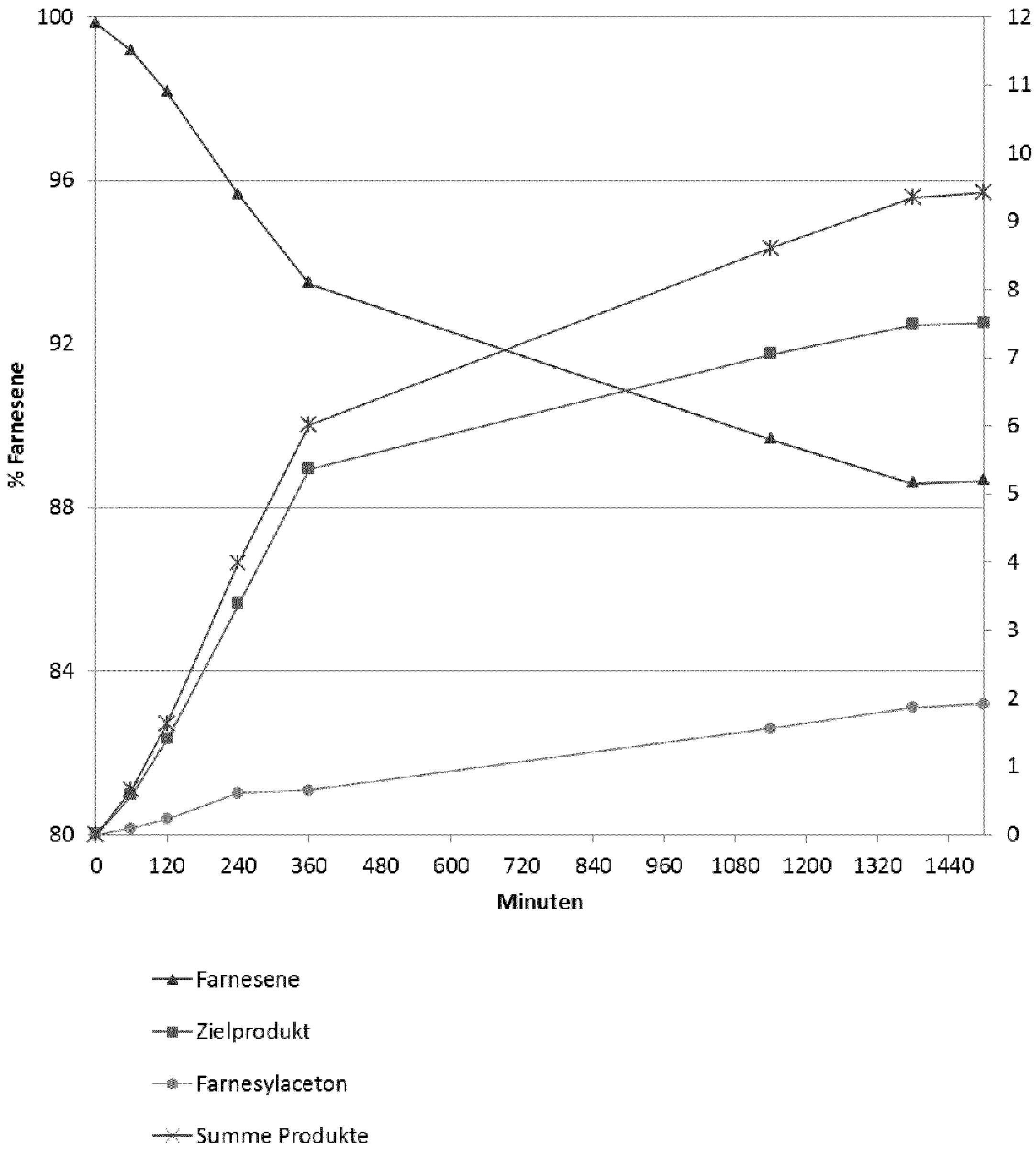
100
96
92
88
84
80
% Farnesene
12
11
10
9
8
7
6
5
4
3
2
1
0
0
120
240
360
480
600
720
840
960
1080
1200
1320
1440
Minuten
Farnesene
Zielprodukt
Farnesylaceton
Summe Produkte

Fig. B4:

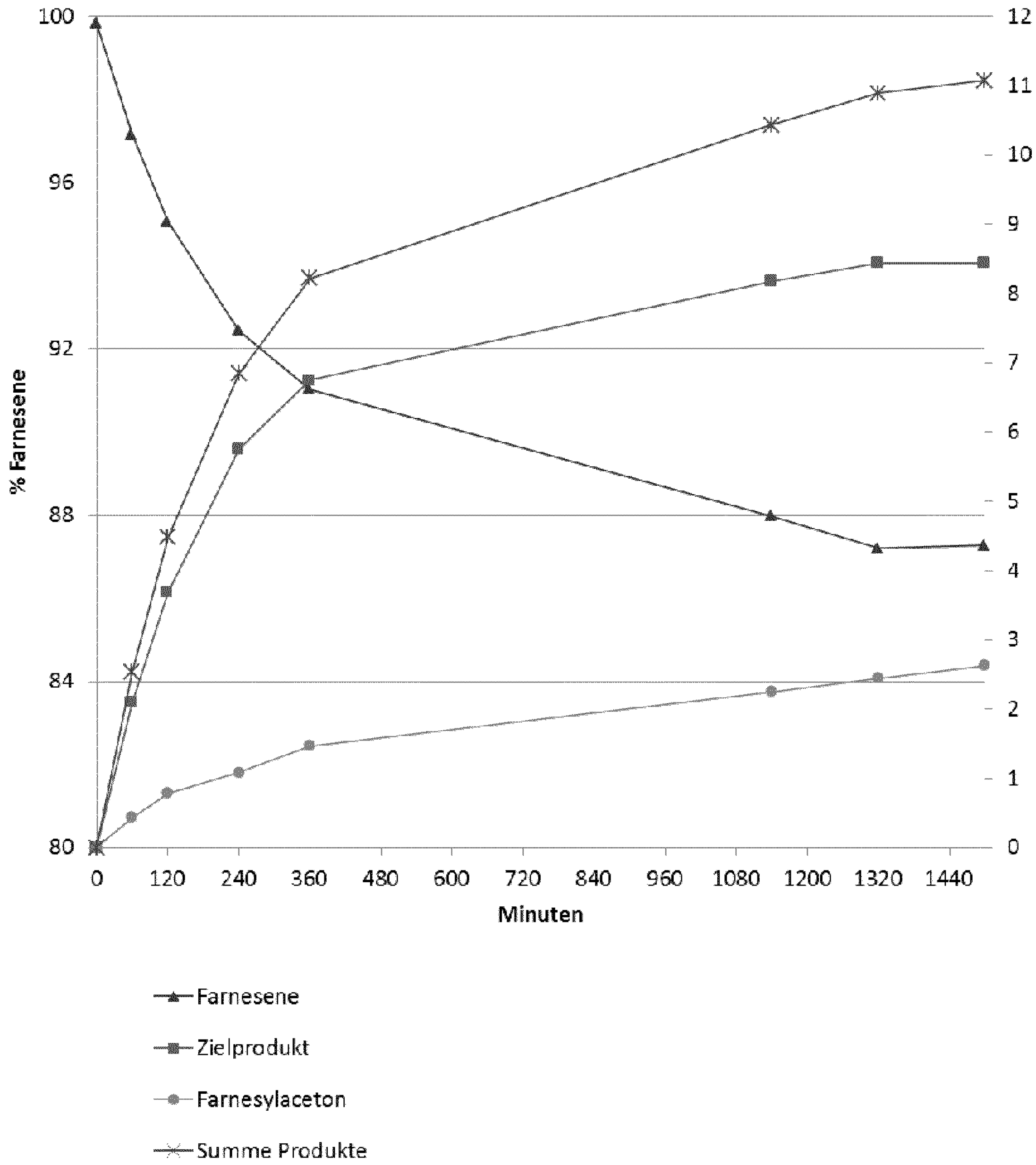
% Farnesene
Minuten
100
96
92
88
84
80
0
120
240
360
480
600
720
840
960
1080
1200
1320
1440
12
11
10
9
8
7
6
5
4
3
2
1
0
Farnesene
Zielprodukt
Farnesylaceton
Summe Produkte

Fig. C1:

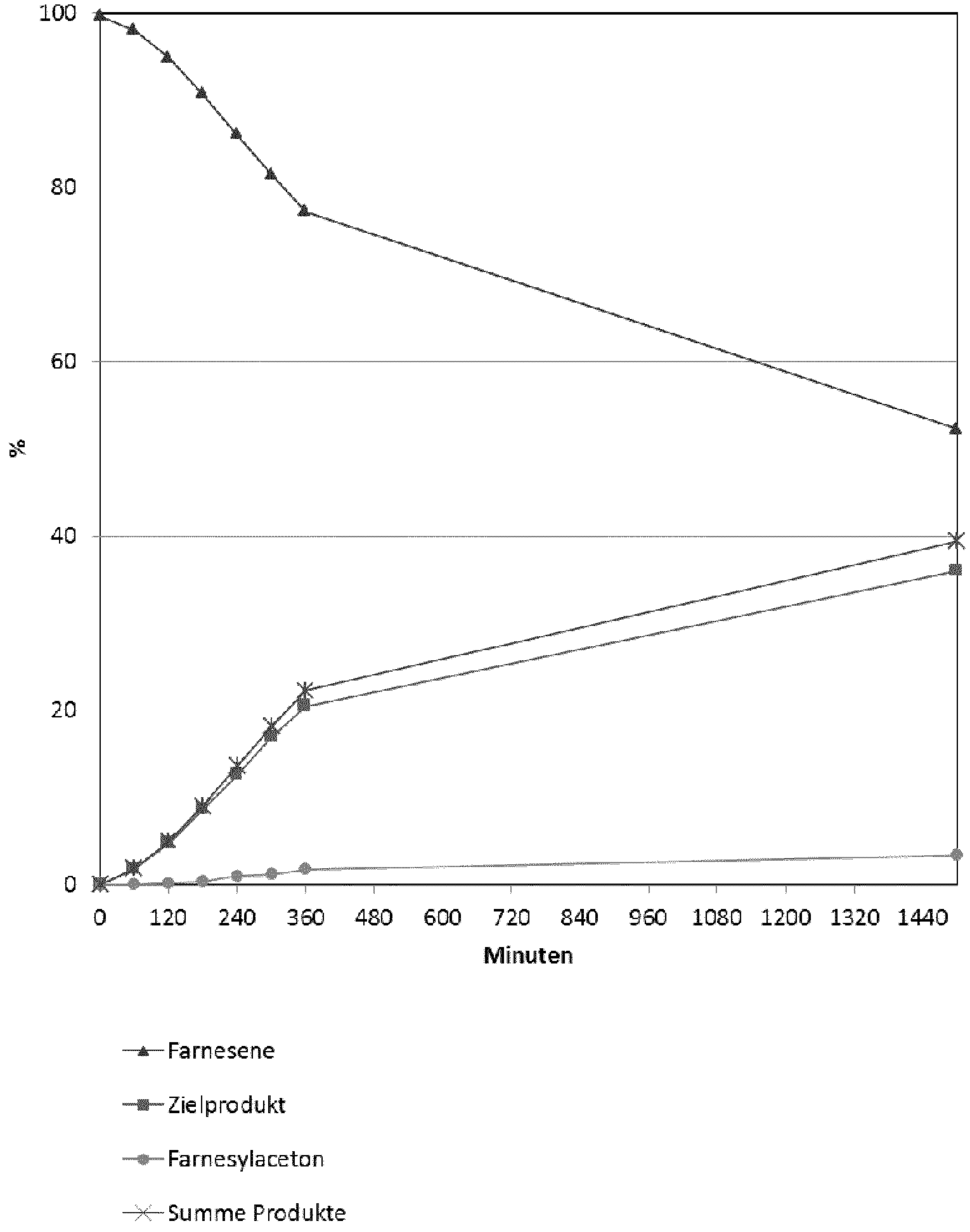
100
80
60
40
20
0
%
0 120 240 360 480 600 720 840 960 1080 1200 1320 1440
Minuten
Farnesene
Zielprodukt
Farnesylaceton
Summe Produkte

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 69103175 T2
- EP 0034804 A2
- WO 2010046199 A
- US 4621165 A
- US 4460786 A
- EP 0441708 A
- EP 34804 A